# EUROPEAN PATENT APPLICATION

(11) **EP 2 071 030 A2**
(43) Date of publication of application: **17.06.2009**
(21) Application number: 09003756.5
(22) Date of filing: 23.01.2004
(51) Int. Cl.: C12N 15/11, A61K 31/7105, A61P 31/14, A61K 48/00

(54) **Oligoribonucleotide or peptide nucleic acid which inhibits action of hepatitis C virus**

(30) Priority: 24.01.2003 JP 2003016750
(62) Divisional of application: 04704739.4
(71) Applicant: Tokyo Metropolitan Organization for Medical Research, Shinjuku-ku, Tokyo 163-8001 (JP); Chugai Seiyaku Kabushiki Kaisha, Kita-ku Tokyo 115-8543 (JP)
(72) Inventor: Kohara, Michinori, Tokyo 1140014 (JP); Watanabe, Tsunamasa, Tokyo 1420041 (JP); Taira, Kazunari, Tsukuba-shi Ibaraki 3050046 (JP); Miyagishi, Makoto, 121-1, Abiko Abiko-shi Chiba 2701166 (JP); Sudo, Masayuki, Kamakura-shi Kanagawa 247-8530 (JP)
(74) Representative: Williams, Aylsa

(57) **Abstract**

A method of inhibiting the replication ability of a hepatitis C virus (HCV) provided. An oligoribonucleotide or a peptide nucleic acid which sequence-specifically binds to the HCV-RNA, and a therapeutic agent for bepatitis C which contains any of these components as an active ingredient are provided.

## Description

### Technical Field

The present invention relates to an oligoribonucleotide or a peptide nucleic acid which inhibits action of a hepatitis C virus, a vector which expresses the oligonucleotide, a therapeutic agent for hepatitis C which contains any of these components as an active ingredient and a method of inhibiting the replication ability of a hepatitis C virus by allowing the oligoribonucleotide or peptide nucleic acid to bind to RNA of a hepatitis C virus.

### Background Art

Hepatitis C virus (hereinafter referred to as "HCV") is a virus which is a main cause of non-A non-B hepatitis after transfusion, and the cDNA of a gene thereof was cloned in 1989. Many researches have been conducted on HCV using a cloned gene cDNA until now, and particularly socially important achievements such as infection prevention and establishment of a diagnostic method have been resulted, leading to a situation at present where HCV infection after transfusion is scarcely observed. However, the number of HCV carriers in the world is estimated to reach several % of all the population.

It is known that the hepatitis caused by HCV infection has a feature of becoming chronic over a long period of time, resulting in chronic hepatitis, which may lead to liver cirrhosis and further to hepatic cancer at a very high rate, and therefore, a reliable remedy for hepatitis after HCV infection is an important subject.

Although interferon (IFN) therapy is widely performed for treating chronic hepatitis C, there exist problems that the rate of effect is about 30%, that side effects such as fever are induced highly frequently, and that the drug is expensive. IFN has been examined by type, dosage or administration and its effectiveness is expected to be improved by the development of consensus IFN etc., and treatment by a combined use of IFN and an antiviral agent such as ribavirin has been attempted, but none of them has resulted in a reliable remedy so far.

In the meantime, to suppress expression of a specific gene within an animal cell in a living body, a method of suppressing expression of the target gene using a double stranded RNA corresponding to the target gene has been found recently (Fire A et al., 1998, Nature, vol. 391, p.806-811). This method is called RNA interference (RNAi) and refers to a phenomenon that when a donble-stranded RNA (dsRNA) is introduced in a cell, mRNA in the cell corresponding to the RNA sequence is specifically decomposed, and the protein encoded by the mRNA is no longer expressed. RNAi is an effective method for investigating the function of a novel gene by preventing gene expression, and is extensively used for functional gene analysis in nematode, drosophila, etc.

However, whether RNAi is effective in the treatment of diseases, particularly viral diseases such as hepatitis C has been unknown.

### Disclosure of the Invention

The present inventors have conducted intensive studies and consequently found that oligoribonucleotides (hereinafter also referred to as "oligo RNAs") or peptide nucleic acids which sequence-specifically bind to the RNA of HCV (HCV-RIVA) inhibit HCV replication, and thus completed the present invention.

That is, the present invention provides the following (1) to (13):
(1) An oligoribonucleotide or peptide nucleic acid which sequence-specifically binds to the RNA (HCV-RNA) of a hepatitis C virus (HCV).
(2) The oligoribonucleotide or peptide nucleic acid according to the above (1) which hybridizes with the RNA of HCV under stringent conditions.
(3) The oligoribonucleotide or peptide nucleic acid according to the above (1) characterized in that the oligoribonucleotide or peptide nucleic acid hybridizes with the sequence of a 5' non-coding region of the RNA of HCV.
(4) The oligoribonucleotide or peptide nucleic acid according to the above (1) characterized in that the oligoribonucleotide or peptide nucleic acid hybridizes with the sequence of a highly identical region of the genetic sequences of a plurality of types of HCV different in genotype.
(5) The oligoribonucleotide or peptide nucleic acid according to the above (1) which is a double-stranded RNA.
(6) The oligoribonucleotide or peptide nucleic acid according to the above (1) which has a chain length of 19 to 23 bp.
(7) An oligoribonucleotide having a nucleotide sequence shown in any one of SEQ ID Nos. 20 to 34.
(8) An oligoribonucleotide which hybridizes under stringent conditions either with an RNA region of HCV having a sequence complementary to the oligoribonucleotide according to above (7) or an RNA region of HCV hybridizing under stringent conditions with said oligoribonucleotide.
(9) An oligoribonucleotide represented by a nucleotide sequence consisting of 19 to 23 contiguous bases in any one of the nucleotide sequences shown in SEQ ID Nos. 47 to 55.
(10) An oligoribonucleotide which hybridizes under stringent conditions either with an RNA region of HCV having a sequence complementary to the oligoribonucleotide according to above (9) or an RNA region of HCV hybridizing under stringent conditions with said oligoribonucleotide.
(11) A vector which expresses the oligoribonucleotide according to any one of above (1) to (10).
(12) A therapeutic agent for hepatitis C containing as an active ingredient the oligoribonucleotide or peptide nucleic acid according to any one of above (1) to (10) or the vector according to above (11).
(13) A method of inhibiting replication ability of HCV by allowing the oligoribonucleotide or peptide nucleic acid according to any one of above (1) to (10) to bind to the HCV-RNA.

The contents described in the specification and/or drawings of the Japanese Patent Application No. 2003-016750 from which the priority is claimed in the present application are incorporated in the present specification.

### Brief Description of the Drawings

Figure 1 shows a general secondary structure in the 5' non-coding region of the HCV-RNA;
Figure 2 shows a general secondary structure in the 3' non-coding region of the HCV-RNA;
Figure 3A shows cDNA sequences of 500 bases from the 5' non-coding region to the core region of the RNAs of HCV-1, HCV-BK and HCV-J which are isolated strain of HCV;
Figure 3B shows cDNA sequences of 500 bases from the 5' non-coding region to the core region of the RNAs of R6, R24 and S 14J which are isolated strains of HCV;
Figure 3C shows cDNA sequences of 500 bases from The 5' non-coding to the core region of the RNAs of HCJ6, JFH1 and ICH1 which are isolated strains of HCV;
Figure 3D shows cDNA sequences of 500 bases from the 5' non-coding region to the core region of the RNAs of JCH3 and HCJ8 which are isolated strains of HCV;
Figure 4A shows a part of the results by multiple alignment of 500 bases from the 5' non-coding region to the core region of various types of HCV;
Figure 4B shows the results (sequel to figure 4A) by multiple alignment of 500 bases from the 5' non-coding region to the core region of various types of HCV;
Figure 4C shows the results (sequel to figure 4B) by multiple alignment of 500 bases from the 5' non-coding region to the core region of various types of HCV;
Figure 5A shows cDNA sequences for the RNA of the 3' non-eoding region of pH77J6S, R6, R24L and R24S;
Figure 5B shows cDNA sequences for the RNA of the 3' non-coding region of HCJ6CH, JFH1, JCH1 and 2b_AB030907;
Figure 6 shows a relation between the addition of siRNA and the amount of HCV core protein produced by Rz-HepM6 cell line;
Figure 7 shows the relation between the addition of siRNA and the activity by which HCV replicon is replicated;
Figure 8 shows the relation between the addition of siRNA prepaid by dicer processing and the activity which HCV replicon replicates; and
Figure 9 shows the relation between the addition of siRNA prepared by dicer processing and the activity which HCV replicon replicates.

### Best Mode for Carrying Out the Invention

The oligo RNA of the present invention which sequence-specifically binds to the HCV-RNA is an oligonucleotide having ribose as sugar and encompasses those containing as a base adenine, guanine, cytosine and uracil which exist in natural RNA, as well as thymine and other modified bases, etc. Although the oligo RNA of the present invention is not particularly limited as far as the oligo RNA can sequence-specifically bind to the HCV-RNA, it is preferable that it is an oligo RNA which inhibits the replication ability of HCV. Examples of the oligo RNA which can sequence-specifically bind to the HCV-RNA include an oligo RNA having a sequence complementary to a sequence of the HCV-RNA, an oligo RNA having a sequence exhibiting a high identity to a sequence complementary to a sequence of the HCV-RNA and an oligo RNA which can hybridize with RNA having a sequence of the HCV-RNA under stringent conditions. In addition, although the present invention is not restrained by any specific theory, it is considered that siRNA, which is one preferable embodiment of the present invention, hybridizes with a target gene within a cell and cleaves the target gene via dicer, and that the target gene is cleaved to the length of 19 to 23 nt. On the other hand, an antisense nucleic acid, which is another embodiment of the present invention, hybridizes with a target gene to induce IFN and activate RNase, thereby decomposing the target gene. Alternatively it is supposed that it binds to the target gene to cause structural change of the target RNA and inhibits translation. Although the sequence of the HCV-RNA includes either of the genomic RNA sequence (- strand) of HCV or the mRNA sequence (+ strand) transcribed from the genomic RNA in the present invention, + strand sequence is more preferred.

In addition, siRNA means an oligo RNA having a length of 19 to 23 nt (19 to 23 bp) in this specification. When the siRNA forms a double strand, one or both of them may have a protruding end.

In the present invention, high identity means identity of 70% or more, preferably identity of 80% or more, and more preferably identity of 90% or more (for example, identity of 95% or more). The algorithm BLAST by Karlin and Altschul (Proc. Natl. Aca. Sci. USA 90:5873-5877, 1993) etc. can be used to determine the identity of base sequences. The programs called BLASTN and BLASTX have been developed based on this algorithm (Altschul et al. J. Mol. Biol. 215:403-410, 1990). When base sequences are analyzed by BLASTN based on BLAST, parameters are set, for example to score = 100 and wordlength = 12. When amino acid sequences are analyzed by BLASTX based on BLAST, parameters are set, for example to score = 50 and wordlength = 3. When BLAST and Gapped BLAST programs are used, default parameters of each program may be used. Specific technique of these analytical methods is well-known (http://www.ncbi.nlm.nih.gov.).

The hybridization technique is a technique well known in the art (for example, Sambrook, J et al., Molecular Cloning 2nd ed., 9.47-9.58, Cold Spring Harbor Lab. press, 1989, etc.) and those skilled in the art can select suitable stringent conditions. Examples of stringent conditions include for example, conditions of 42°C, 5×SSC and 0.1% SDS in the washing after hybridization, preferably conditions of 50°C, 5×SSC and 0.1% SDS, and more preferably conditions of 65°C, 0.1×SSC and 0.1% SDS. However, as for factors which influence the stringency of hybridization) plural factors such as temperature and salt concentration can be considered, and those skilled in the art could select and adjust these factors suitably to achieve the similar stringency.

The oligo RNA of the present invention may be single stranded or double stranded, or may be multiple stranded formed of two or more strands but preferably double stranded. The double strand may be formed of two independent strands or may be a double stranded structure formed in a self-complementary single stranded RNA, which can form a stem-loop structure by one molecule. When an oligo RNA is double stranded, it may be double stranded in all regions or have some regions (for example, both ends or one end, etc.) of other structures such as single strand.

The length of the oligo RNA of the present invention is not limited as long as it has a sequence-specific binding ability to the HCV-RNA. Examples of the length of the oligo RNA of the present invention include 5 to 1000 bases (5 to 1000 bp in the case of double strand), preferably 10 to 100 bases (10 to 100 bp in the case of double strand), more preferably 15 to 25 bases (15 to 25 bp in the case of double strand), and particularly preferably 19 to 23 bases (19 to 23 bp in the case of double strand).

In the present invention, a preferable oligo RNA is an oligo RNA which has a nucleotide sequence shown in SEQ ID Nos. 20 to 34, and particularly preferably an oligo RNA which has a nucleotide sequence shown in SEQ ID Nos. 20 to 25. In addition, the other preferable oligo RNAs in the present invention include oligo RNAs represented by the nucleotide sequence which consists of 19 to 23 contiguous bases in the nucleotide sequence shown in SEQ ID Nos. 47 to 55.

Examples of the still other preferable oligo RNAs in the present invention include an oligo RNA which hybridizes under stringent conditions either with an RNA region of HCV having a sequence complementary to the above oligo RNA having a nucleotide sequence shown in SEQ ID Nos. 20 to 34, or with an RNA region of HCV hybridizing under stringent conditions with said oligo RNA, and an oligo RNA which hybridizes under stringent conditions either with an RNA region of HCV represented by a nucleotide sequence consisting of 19 to 23 contiguous bases in nucleotide sequence shown in SEQ ID Nos. 47 to 55, or with an RNA region of HCV hybridizing under stringent conditions with said oligoribonucleotide. Those skilled in the art could readily determine in any type of HCV an RNA region of HCV which hybridizes under stringent conditions with these RNAs. Examples of these oligo RNAs include a nucleotide sequence described in SEQ ID Nos. 20 to 34 or a nucleotide sequence consisting of 19 to 23 contiguous bases in nucleotide sequences shown SEQ ID Nos. 47 to 55 wherein 7 or less, preferably 5 or less, more preferably 3 or less nucleotides are deleted, substituted or added and which can inhibit HCV replication by hybridizing with the RNA of HCV.

In addition, peptide nucleic acids which can be suitably used in the present invention include peptide nucleic acids having base sequences corresponding to oligo RNAs which can be suitably used in the present invention.

The RNA of HCV consists of a non-coding region on the 5'-end side (5'-end non-coding region) containing about 340 nucleotides, an open reading frame (ORF) of about 9,400 nucleotides, and a non-coding region on the 3'-end side (3'-end non-coding region) containing about 50 nucleotides. Although a site targeted by the oligo RNA of the present invention is not particularly limited but may be any site of the RNA sequence, the target site is preferably located from the 5' non-coding region to the 5'-end region of ORF (for example, regions having base sequences shown in SEQ ID Nos. 1 to 11) or in the 3' non-coding region (for example, regions having base sequences shown in SEQ ID Nos. 12 to 19), more preferably the 5'-end non-coding region.

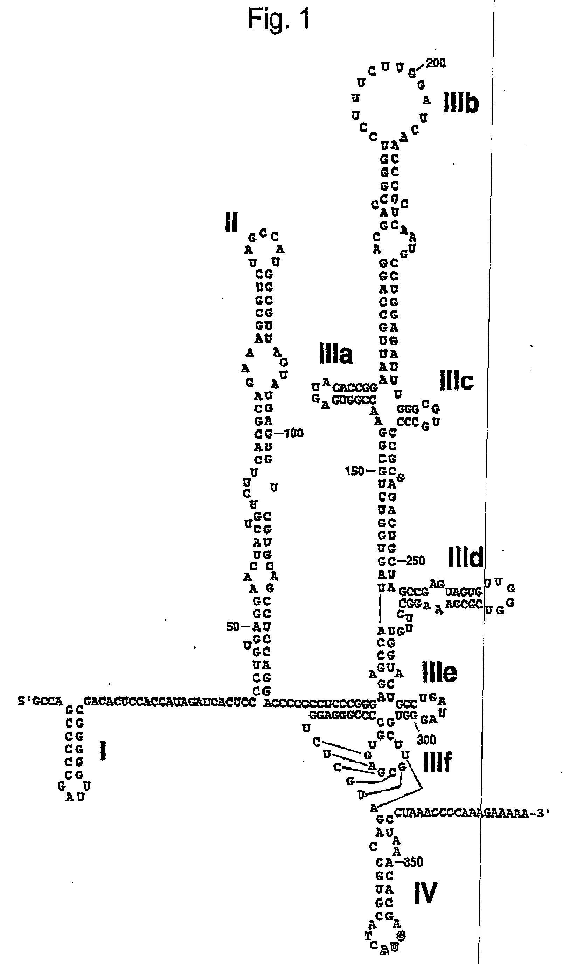

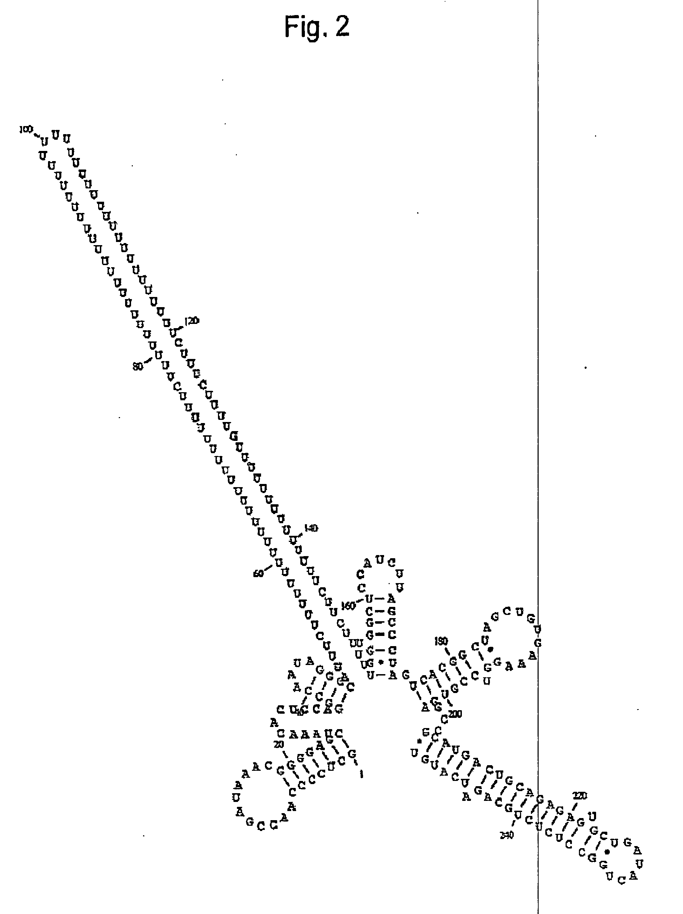

An internal ribosomal entry site (IRES) and stem regions which form a stem loop are present in the 5' non-coding region of the HCV-RNA. There have been many reports on the 5'-end non-coding region, IRES and the stem region for HCV (Kato. N. et al., Proc Natl. Acad. Sci. USA., 87, 9524-9528 (1990), Proc. Natal. Acad. Sci. USA., 88, 2451-2455 (1991), J. Viol., 65, 1105-1113 (1991), J. Gen. Viol., 72, 2697-2704 (1991), Virology, 188, 331-341 (1992), Tsukiyama. Kohara et al., J. Virol., 66, 1476-1483 (1992), Honda Masao et al., J. Virol., 73, 1165-1174 (1999), Honda Masao et al., RNA, 2 (10), 955-968 (1996), Sasano T. et al., Genome Inf. Ser., 9, 395-396 (1998), Ito T et al., J. Virol., 72, 8789-8796 (1998), Kamoshita N et al., Virology., 233, 9-18 (1997), etc. General secondary structures in the 5 non-coding region and 3' non-coding region of the HCV-RNA are shown in Figures 1 and 2.

As for HCV, there are plural types of HCV which are different from each other in genotype. Examples thereof include HCJ6, HCJ8, HCV-1, HCV-BK, HCV-J, JCH1, JCH3, JFH1, R24, R6, S14J, pH77J6S (GenBank Accession no. AF177039), HCJ6CH, 2 b_AB030907, etc. In order to deal with these plural HCV-RNAs which are different from each other in genotype, it is preferable to target a region having a high identity among the plural HCV gene sequences which are different from each other in genotype. The region having a high identity among the plural HCV gene sequences which are different from each other in genotype as used herein means a region where the RNA sequences of plural types of HCV have an identity of 80% or more, preferably an identity of 90% or more, more preferably an identity of 95% or more. Such a region preferably has a length of 10 or more bases, more preferably a length of 15 or more bases, and particularly preferably a length of 20 or more bases. Plural types of HCV as used herein usually refer to 3 or more types of HCV, preferably 5 or types of HCV and particularly preferably 10 types of HCV. The identity of the gene sequences can be calculated by comparing of the plural types of the object genes and applying the above-mentioned algorithm and the like.

There are no particular limitation on the oligoribonucleotide used in the present invention, and in addition to those having normal RNA constructs which are no modified, modified RNAs having a phosphate diester moiety or a sugar moiety modified can be used. Moreover, the oligo RNA of the present invention may contain as its portion a non-ribonucleotide molecule such as deoxyribonucleotide.

In addition, a peptide nucleic acid (PNA) etc. can be used instead of an oligo RNA in the present invention. PNA is a technique well known in the art (Nielsen Peter E., Methods in Molecular Biology, 208, 3-26 (2002); Braasch Dwaine A et al., Biochemistry, 41 (14), 4503-4510 (2002); Koppelbus Uffe et al., Antisense Drug Technology, 359-174 (2001); Nielsen Peter E., Methods in Enzymology, 340, 329-340 (2001)) and like the above-mentioned oligo RNA, a PNA which can sequence-specifeally bind to the HCV-RNA can be prepared. The length of a suitable peptide nucleic acid in the present invention is, for example, 5 to 1,000 bases (5 to 1,000 bp in the case of double strand), preferably 10 to 100 bases (10 to 100 bp in the case of double strand), more preferably 15 to 25 bases (15 to 25 bp in the case of double strand) and particularly preferably 19 to 23 bases (19 to 23 bp in the case of double strand).

The oligo RNA or peptide nucleic acid of the present invention can be repared by well-known methods to those skilled in the art.

When the oligo RNA, of the present invention is to be expressed continuously, a vector which expresses the oligo RNA of the present invention may be prepared. A vector can be prepared by well-known methods to those skilled in the art. For example, it can be prepared by introducing a gene encoding the oligo RNA of the present invention into a well-known vector such as those described in Nature Biotech (2002) 19, 497-500. Promoters suitable for the expression of the oligo RNA of the present invention are not particularly and examples thereof include T7 promoter, tRNA promoter, U6 promoter, etc.

Since the oligo RNA of the present invention can inhibit HCV replication and suppress HCV multiplication, it is useful as a therapeutic agent for hepatitis C. In this when an oligoribonucleotide or peptide nucleic acid corresponding to plural types of HCV is provided, treatment can be effected without clinically identifying the type of a virus with which the patient is infected and it is not necessary to use a combination of plural types of oligoribonucleotide or peptide nucleic acid and therefore, such an embodiment is preferable.

When the drug is used for medical treatment, it can also be administered in the form which can function within a cell as it is. In this case, about 19 to 23 bases are optimal as the length of the oligo RNA or peptide nucleic acid. Moreover, the drug can also be administered in the form which can function through processing in a cell. In this case, the oligo RNA or peptide nucleic acid which has a longer sequence than the sequence including the desired sequence can be administered. The double-stranded RNA (dsRNA) taken into the cell is decomposed to about 21mer by an enzyme called dicer, and serves as siRNA (short-interfering RNA), and forms a complex called RISC (RNA-Induced Silencing Complex) which is supposed to destroy RNA having a specific base sequence transcribed from the genome (Bernstein, E. et al., Nature, 409:363-366, 2001; Hammond, S. M. et, al., Nature, 404:293-296, 2000). Alternatively, siRNA prepared beforehand *in vitro* using a commercially available dicer can also be used.

The therapeutic agent for hepatitis C comprising the oligo RNA or the peptide nucleic acid of the present invention as an active ingredient can be prepared by adding pharmaceutically acceptable excipients, tonicity agent, dissolution auxiliary agent, stabilizing agent, antiseptic, soothing agent, etc. if needed, to form a pharmaceutical composition such as a tablet, powder, granule, capsule, liposome capsule, injection, liquid and nasal drop, and can be further a lyophilized agent. These can be prepared according to usual methods. In addition, it is also possible to administer a vector which expresses the oligo RNA of the present invention.

Although the administration route of the oligo RNA or peptide nucleic acid of the present invention is not particularly limited, it is applied to a patient so that it may finally reach the affected site, preferably for example, by directly applying it to the affected site of the patient or administering it into a blood vessel. Furthermore, an enclosure material which enhances durability and membrane permeability can also be used. Examples thereof include liposome, poly-L-lysine, lipid, cholesterol, lipofectin and a derivative thereof.

The dosage of the oligo RNA or peptide nucleic acid of the present invention can be suitably adjusted according to the patient's condition to provide a preferable amount. For example, it can be administered in a range of 0.001 to 100 mg/kg, preferably 0.1 to 10 mg/kg, but it is not particularly limited.

Furthermore, the present invention provides a method of inhibiting the replication ability of HCV by allowing the above-mentioned oligoribonucleotide or peptide nucleic acid of the present invention to bind to the RNA of HCV. The method of the present invention includes contacting a sample, which contains or may contain HCV, with an oligo RNA or peptide nucleic acid of the present invention either in vivo or *in vitro.* The result of prevention of the replication ability of HCV can be detected by methods usually used in the art.

The present invention is described further referring to Examples hereinbelow, but the present invention is not limited to these Examples.

### Example 1: Determination of the region to be targeted by siRNA

Sequences were compared in order to find regions having a high homology in the base sequence which constitutes the gene in many isolated strains of hepatitis C virus, particularly from 5' non-coding region to the core region and in 3'-end non-coding region.

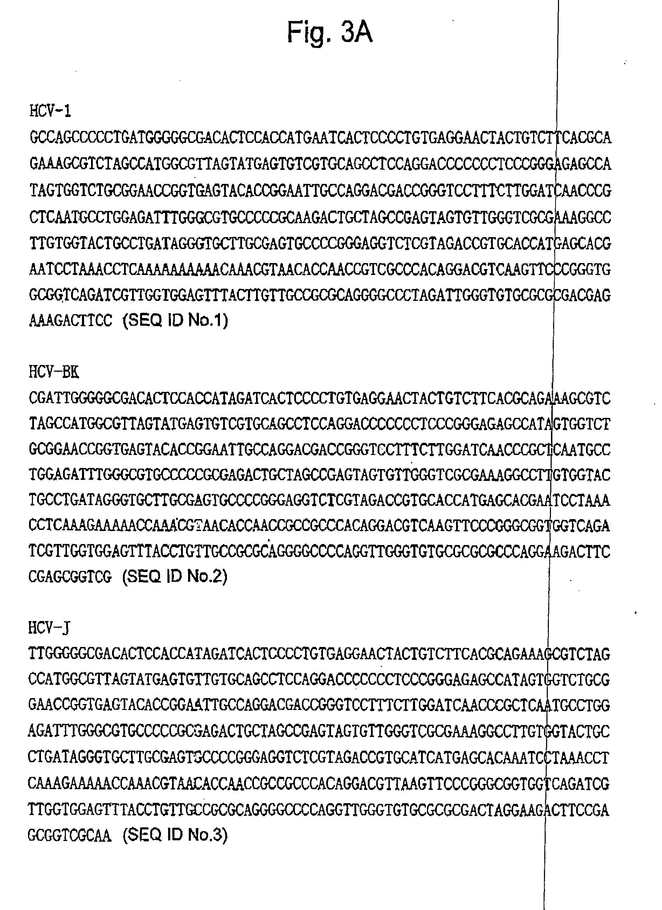

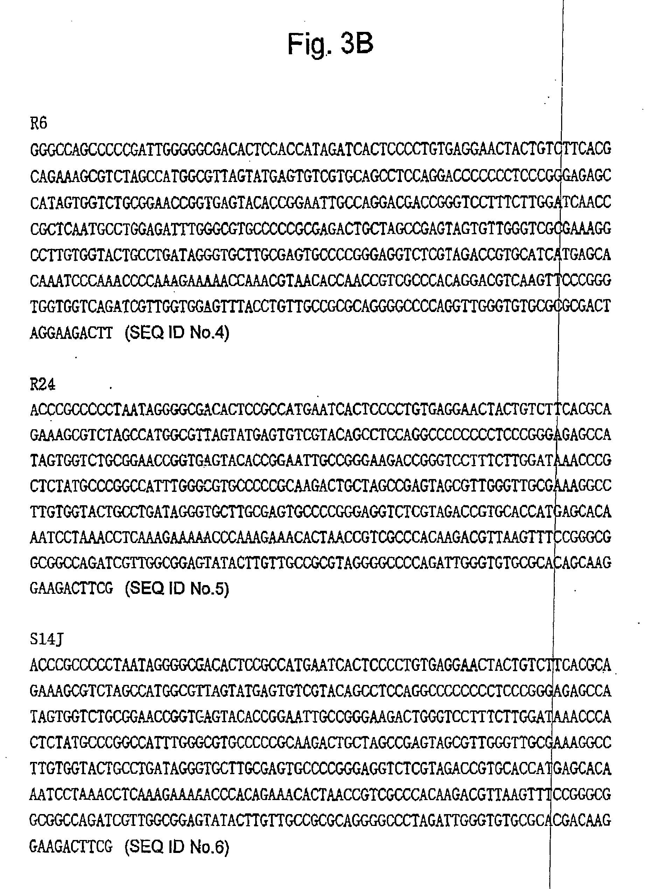

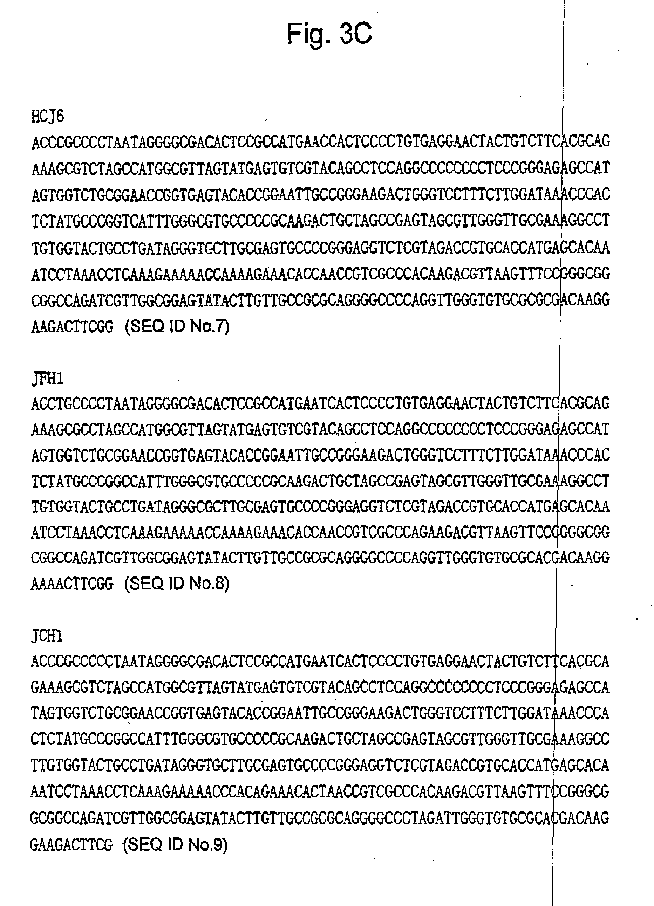

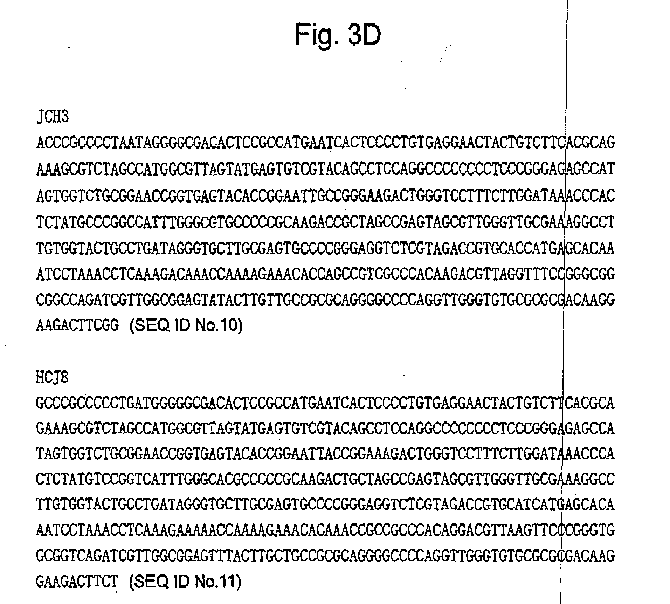

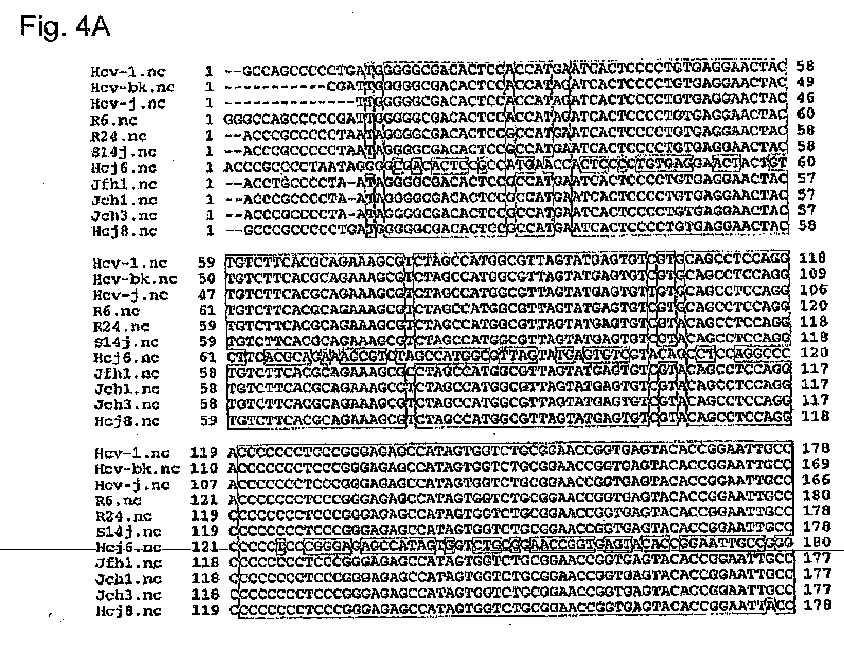

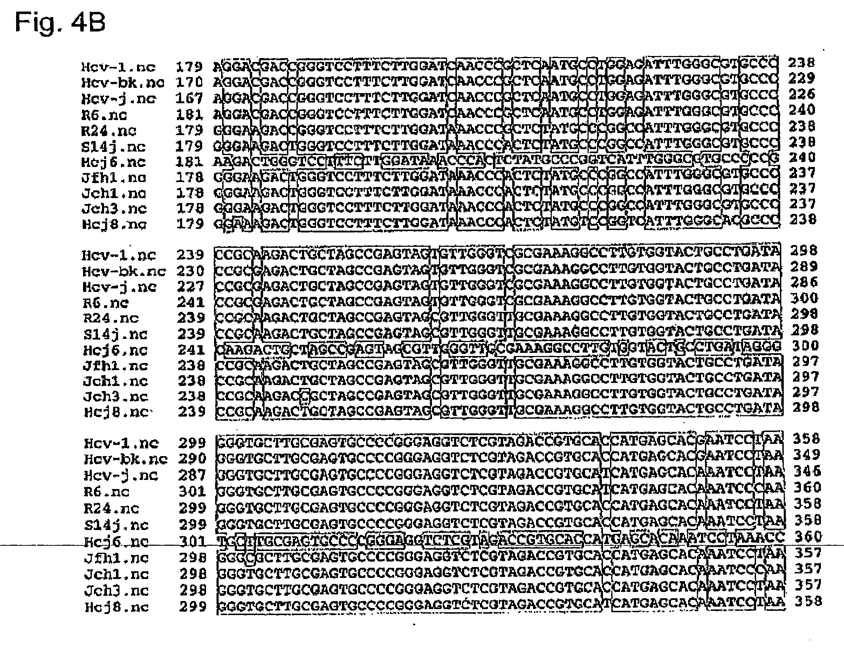

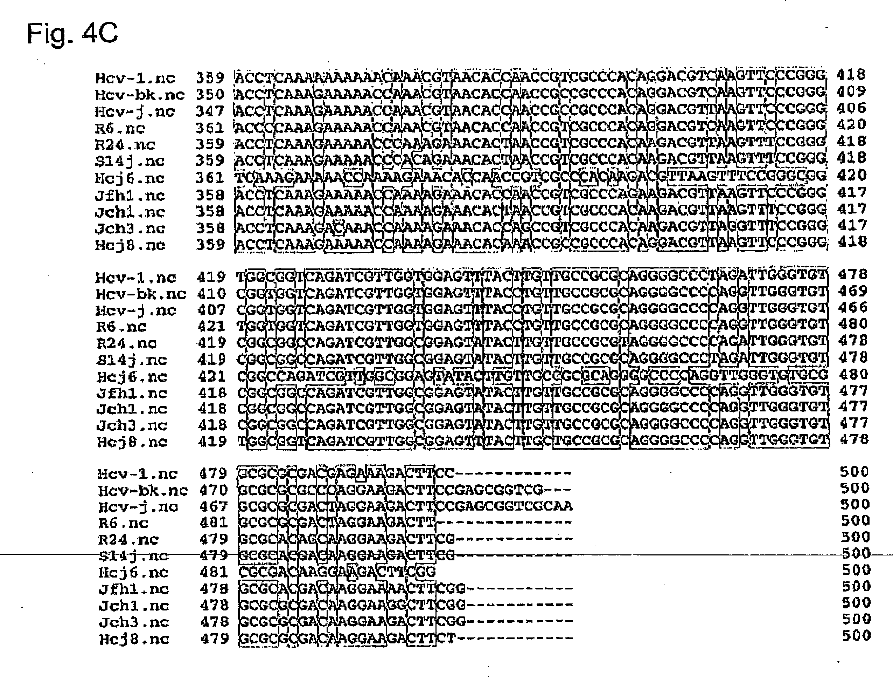

The cDNA sequences of about 500 bases from 5' non-coding region to the core region of the RNA of HCV-1 (GenBank Accession no. M62321), HCV-BK (Accession no. M58335), HCV-1 (Accession no. D90208), R6 (Accession no. AY045702), R24, S14J, HCJ6, JFH1 (Accession no. AB047639), JCH1 (Accession no. AB047640), JCH3 (Accession no. AB047642), HCJ8 (Accession no. D10988) which are isolated strains of HCV are shown in Figure 3. Multiple alignment was performed using the method usually performed in the art for these base sequences. The results are shown in Figure 4. Based on the region on the 5'-end side of HCV in which at least 10 types of HCV have an identity of 95% or more, siRNA which can sequence-specifically bind to this region as the target was designed.

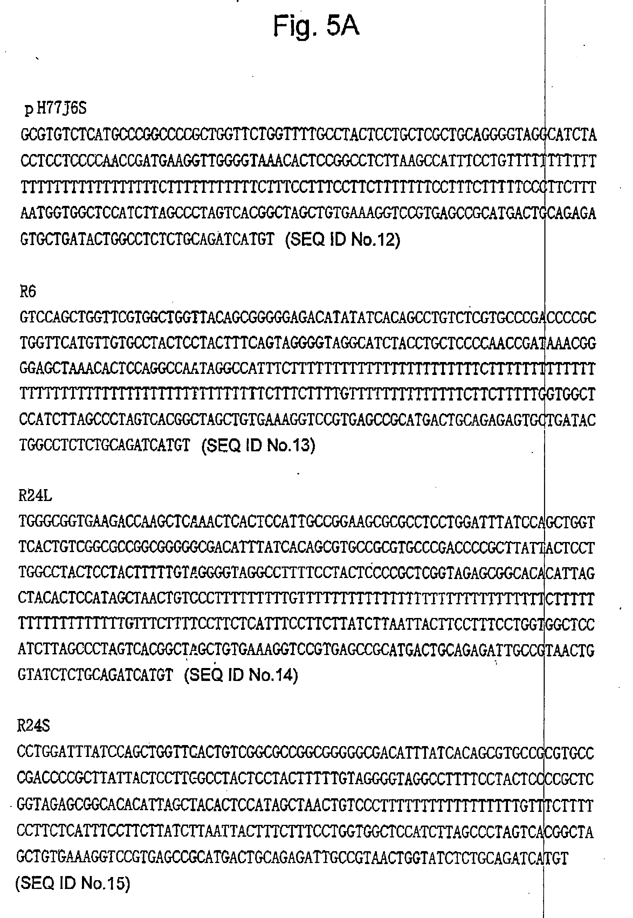

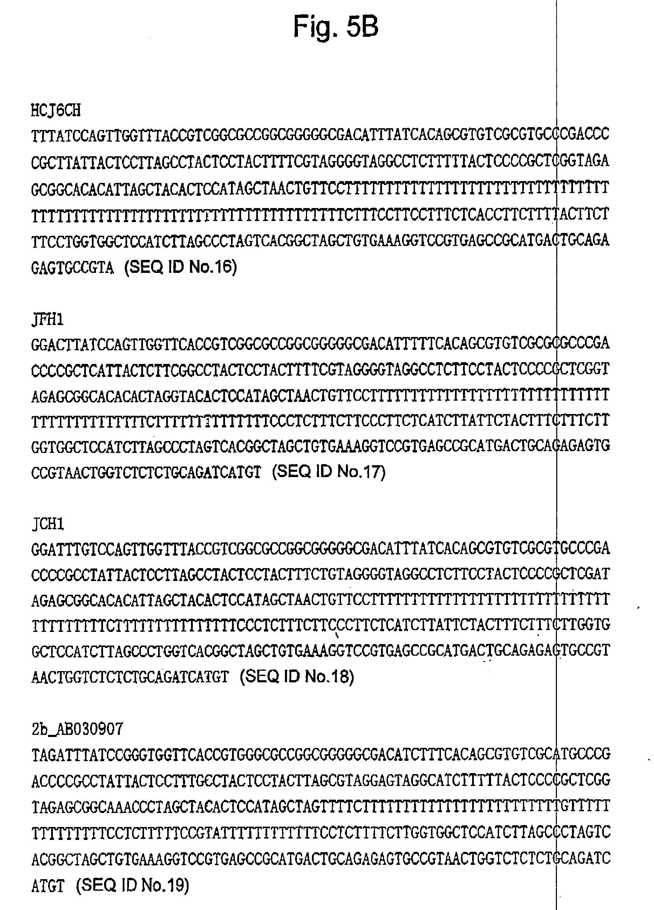

Multiple alignment was similarly carded out for the non-coding region on the 3'-end side using the sequences of pH77J6S (Accession no. AF177039), R6, R24L, R24S, HCJ6CH (Accession no. AF177036), JFH1, JCH1 and 2 b_AB030907 (Accession no. AB030907) which were similarly shown in Figure 5, and based on the region in which at least 8 types of HCV have an identity of 95% or more, siRNA which can sequence-specifically bind to this region as the target was designed.

In the meantime, it is preferable to design siRNA in consideration of the above-mentioned sequence identity, and in consideration that it may be bound to the region just before the translation initiation site. Furthermore, it is preferable to design also in consideration of the secondary structure of HCV RNA which serves as the target. Particularly, 5'- and 3'-UTR loop structure and their neighboring regions can be used as a target.

### Example 2: Synthesis of siRNA

Based on the results of Example 1, a sequence of siRNA having a length of 21 nt was designed to all the target HCV genomes, and an oligonucleotide which includes T7 promoter sequence in the 3'-end was synthesized according to the protocol of Silencer siRNA Construction Kit (Ambion cat. no. 1620). 100 µM of each oligonucleotide used as a template was prepared, allowed to hybridize with T7 primer, and converted to a double-stranded DNA using Klenow enzyme and transcribed using T7 promoter. After carrying out annealing of the synthesized RNA to each complementary strands to form a double-stranded RNA, the remaining single stranded protruding ends were digested by RNase to prepare a siRNA. 15 to 30 µg/reaction of siRNA firally synthesized was adjusted to 10 µM with a RNase free water, and after the presence of the double-stranded RNA of 20 to 22 bases was confirmed by 12% acrylamide gel electrophoresis, it was stored at -80°C until it was used.

The synthesized siRNA sequences were shown below. The nucleotide numbers in the sequence of HCV (R6 strain) (Accession no. AY045702, SEQ ID No. 56) to which these sequences correspond are also indicated.
1) siRNA targeting 5'-UTR
   R1-siRNA; 5-GGAACUACUGUCUUCACGCAG-3'(21 bases) (SEQ ID No. 20, 53-73nt)
   R2-siRNA; 5'-GCCAUAGUGGUCUGCGGAACC-3'(21 bases) (SEQ ID No. 21, 139-159nt)
   R3-siRNA; 5'-AGGCCUUGUGGUACUGCCUGAU-3'(22 bases) (SEQ ID No. 22, 278-299nt)
   R5-siRNA; 5'-GUCUCGUAGACCGUGCAUCA-3'(20 bases) (SEQ ID No. 23, 325-344nt)
   R6-siRNA; 5'-GCGAAAGGCCTTGTGGTACTG-3'(21 bases) (SEQ ID No. 24, 273-293nt)
   R7-siRNA; 5'-GTCTCGTAGACCGTGCACCA-3'(20 bases) (SEQ ID No. 25, 325-344nt)
   R5L-siRNA; 5'-GUCUCGUAGACCCGUGCAUCAT-3'(21 bases) (SEQ ID No. 26, 325-345nt)
   R1mut-siRNA; 5'-GGAACUACUGUCUUCACGCAG-3'(21 bases) (SEQ ID No. 27, 53-73nt)
   R2mut-siRNA; 5'-GCCAUAGUGGUCUGCCGAACC-3'(21 bases) (SEQ ID No. 28, 139-159nt)
   R3mut-siRNA; 5'-AGGCCUUGUGGUACUGCCUGAU-3'(22 bases) (SEQ ID No. 29, 278-299nt)
   R5mut-siRNA; 5'-GUCUCGUAGACCGUGCAUCA-3'(20 bases) (SEQ ID No. 30, 325-344nt)
   R6mut-siRNA; 5'-GCGAAAGGCCTTGTGGTACTG-3'(21 bases) (SEQ ID No. 31, 273-293nt)
   R7mut-siRNA; 5'-GTCTCGTAGACCGTGCACCA-3'(20 bases) (SEQ ID No. 32, 325-344nt)
2) siRNA targeting 3'-UTR
   R8-siRNA; 5'-GGCTCCATCTTAGCCCTAGTC-3'(21 bases) (SEQ ID No. 33, 9515-9535nt)
   R9-siRNA; 5'-GGCTAGCTGTGAAAGGTCCGT-3'(21 bases) (SEQ ID No. 34, 9538-9558nt)

### Example 3: Effect on the expression of the core protein of HCV

The present inventors have already established a system in which switching expression of all the HCV genomes are carried out by Cre/loxP system (J. Biol. Chem., 273, 9001-6. (1998)). The present inventors have established a human origin liver cell line Rz-HepM6 which carries out self-sustaining expression of all the HCV genomes (Genotype 1b, nucleotide no. 1-9611nt) using Cre recombinase this time, and this was used as the target. The Rz-HepM6 cell was suspended to DuIbecco's Modified Eagle medium (NISSUI cat. no. 05915) which contains 10% fetal bovine serum (REHATUIN cat. no. 1020-90), and seeded on a 24-well plate in 10⁵ cells per well, and cultured overnight at 37°C under 5% CO₂. siRNA was introduced when the cell density was 50 to 70%. That is, 2.0 µl of Oligofectamine transfection reagent (Invitrogen cat. No. 12252-011) and 5.5 µl of Opti-MEM1 (Gibco cat. No. 22600) was added and mixed well, and it was allowed to stand still for 10 minutes at room temperature. Then, 5.0 µl of synthesize 10 µM siRNA was diluted in 40 µl of Opti-MEM1 and added so that the final concentration might be 200 nM. After allowed to stand still for 20 minutes at room temperature, siRNA to which the Oligofectamine reagent was added was directly added to the cell for which the culture solution was exchanged to 200 µl of Opti-MEM1 beforehand and cultured at 37°C under 5%CO₂.

In 4 hours after siRNA was added, 125 µl of Dulbecco's Modified Eagle medium containing 30% fetal bovine serum, which was three times in the concentration, was added, and cultured at 37°C under 5%CO₂. Cells were collected in 24 hours after the serum was added with 20 µl of lysis buffer (1% SDS, 0.5% NP40, 0.15M NaCl, 0.5 mM EDTA, 1 mM DTT, 10 mM Tris:pH7.4), and quantification of the HCV core protein was carried out using a HCV core quantification kit (International Reagents cat No. 14861).

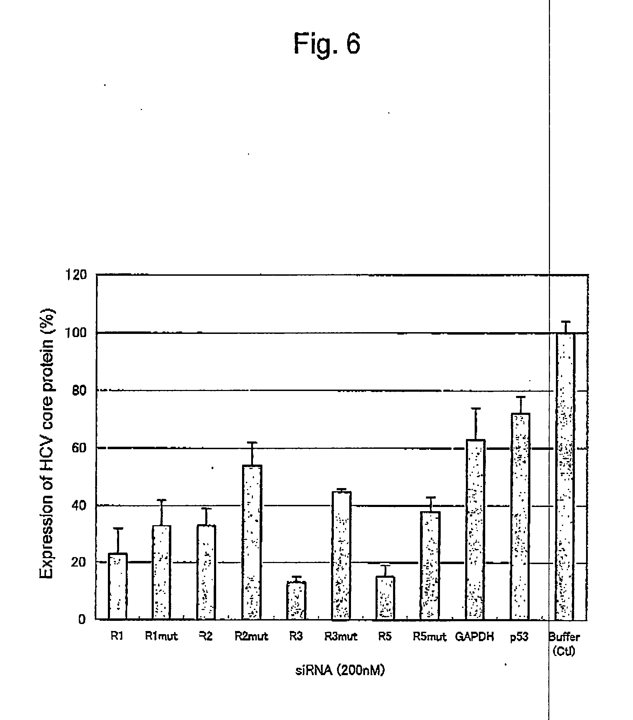

The relation between the addition of siRNA and the amount of HCV core protein which Rz-HepM6 cell line produces is shown in Figure 6. The quantification of the core protein which constitutes a virus particle was carried out by ELISA method after adding 200 µM each of siRNA (R1-siRNA, R2-siRNA, R3-siRNA, R5-siRNA, R1mut-siRNA, R2mut-siRNA, R3mut-siRNA and R5mut-siRNA). Although all of the added siRNAs inhibited synthesis of the core protein, it was observed that particularly the action of R3 and R5 was strong, and the specificity of their base sequences was also high. Furthermore, inhibitory effect of the expression of the core protein by R3mut and R5mut, sequences into which variation was introduced, was decreased.

### Example 4: Replicon assay

In order to carry out the quantification of the number of copies of the HCV-RNA, those having a luciferase gene derived from firefly as a reporter gene introduced into the HCV-RNA were constructed. According to the method by Krieger et aL (J. Virol., 75, 4614 24 (2001)), the luciferase gene was introduced immediately after the Internal Ribosome Entry Site (IRES) of HCV gene in the form a fused gene with neomycin resistance gene. After the RNA of interest was synthesized *in vitro*, it was introduced into Huh7 cell (Japanese Collection of Research Bioresources) by electroporation method, and isolated as a G418 resistance clone. The firefly luciferase HCV replicon cell (Huh-3-1) was suspended to Dulbecco MEM (Gibco cat. no. 10569) which contains 5% fetal bovine serum (Hyclone cat. no. SH 30071.03), and 5000 cells per well were seeded on a 96-well plate and cultured overnight at 37°C under 5%CO₂. In about 20 hours, diluted siRNA was added in an amount of 10 µl per well, and was cultured for three more days. The assay plate was prepared in two lines, one assay was performed on the white plate and the other was performed on the clear plate.

The white plate was used for Steady-Glo Luciferase Assay System (Promega cat. no. E2520) after the culturing was finished. That is, 100 µl of the reagent per well was put in, mixed with a pipette 3 to 4 times, and luminescence was measured by 1450 MicroBeta TRILUX (WALLAC) after allowing it to stand still for 5 minutes.

The synthesized siRNAs were introduced into the HCV replicon cells by the following methods. That is, 10000 cells per well were seeded on a 96-well plate and cultured overnight at 37°C under 5%CO₂, siRNA was introduced when the cell density was 50 to 70%. That is, 1.5 µl of TransIT-TKO transfection reagent (Mirus Corporation cat. No. MIR2150) and 25 µl of Opti-MEM1 (cata no. 31985) were vigorously agitated and then allowed to stand still for 20 minutes. 0.125 to 1.25 µl of siRNA was mixed and they were gently stirred and allowed to stand still for further 20 minutes. This solution was calmly added to 100 µl of the cell in 96-well plate, and cultured overnight at 37°C under 5%CO₂, Replicon assay was performed using these cells. siRNA was added so that the final concentration might be 1 nM 10 nM, 30 nM and 100 nM and introduced with TransIT-TKO transfection reagent, and in 24 hours, HCV replicon activity was measured using the reporter gene (luciferase activity) as an index. The activity of each siRNA was calculated by subtracting the value in which the cells were not added as a background from all the values and assuming the activity when no siRNA was added as 100%.

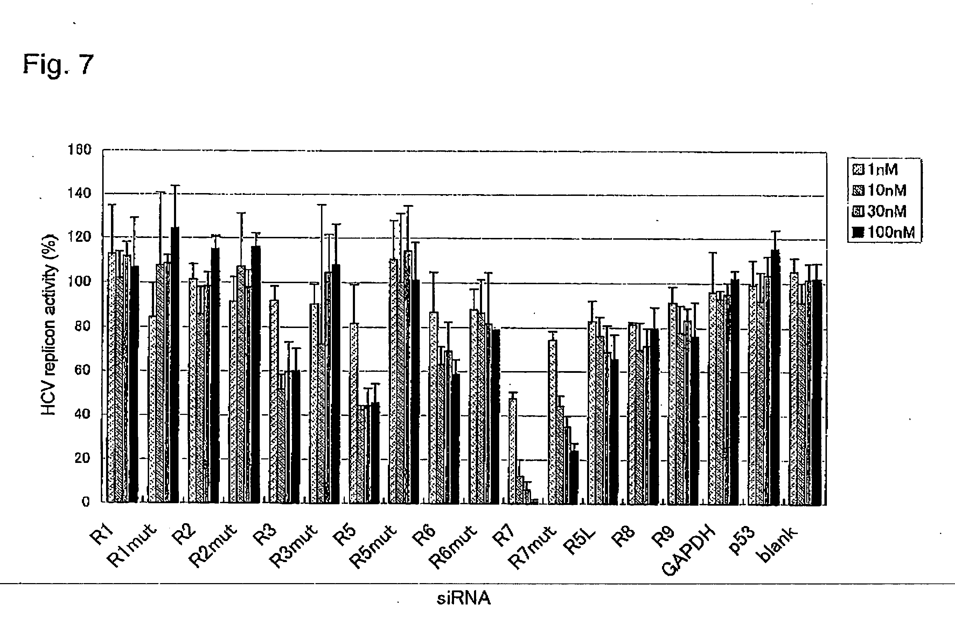

The relation between the addition of siRNA and the activity by which HCV replicon is replicated is shown in Figure 7. Sequences R3, R5, R6 and R7 inhibited the activity of replicon in dose dependence. Sequence R3mut, R5mut, R6mut and R7mut in which the base sequences are partially substituted have decreased effect and therefore it is considered that the sequences R3, R5, R6 and R7 exhibit sequence-specific antivirotic effect.

### Example 5: Calculation of RNA transfection efficiency

siRNA was labeled with Cy3 using Silencer siRNA Labeling Kit (Ambion cat no. 1632) according to the protocol. That is, of 7.5 µl of Cy3 labeling reagent was added to 10 µM (19.2 µl) of R7-siRNA, and the labeling was performed at 37°C in a shaded condition in 50 µl for 1 hour. 5 µl of 5M NaCl and 99.5% ethanol 2.5 times in volume were added and ethanol precipitation was performed at -20°C. The Cy3-labeled siRNAs were collected by centrifugation at 4°C, 15000 rpm. The quantification of the labeled siRNA was carried out by calculating from the maximum absorption and the molecular extinction coefficient of Cy3 http://www.ambion.com/techlib/append/base_dye.html).

The obtained Cy3-labeled siRNAs were introduced into the cells using the TransIT-TKO transfection reagent, and were observed with fluorescence microscope in 24 hours. After confirming the positions of the cells in the view of phase-contrast microscope at first, the cells dyed with Cy3 were observed with fluorescence microscope. The wavelength used at this time was 510 nm for the exciting wavelength and 550 nm for the absorption wavelength. It became clear that the Cy3-labeled cells were about 90% of the whole cells, which was very high transfection efficiency.

### Example 6: Preparation of si-RNA by dicer

HCV R6 gene (Accession no. AY045702, SEQ ID No. 56) was used as a template, each of the combinations shown in Tables 1 and 2 as primers and PCR reaction was performed following the normal method. Primers were designed by selecting regions in which homology among plural types of HCV exists or regions important for the replicative function of HCV. After excising and purifying the obtained PCR product from the gel, transcription reaction (20 µl vol × 4 hours) was performed using T7 RNA polymerase (for example, MEGAscript T7, Ambion Inc. cat # 1334), RNA was synthesized, and the double-stranded RNAs of having sizes of the object RNAs were confirmed by agarose gel electrophoresis (precursor siRNA-1 to precursor siRNA-9, SEQ ID Nos. 47 to 55). Subsequently, DNaseI is used and after reacting for 15 minutes, LiCl precipitation was carried out, and the product was dissolved in 20 µl of Nuclease free Water, and the amount of RNA was measured by absorption (total about 30 to 60 µg of dsRNA/reaction).

**Table 1**

| Designation of double-stranded RNA | Primer 1 | Primer 2 |
|---|---|---|
| Precursor siRNA-1 | Ds5-41-S25 | Ds5-612-R23 |
| Precursor siRNA-2 | Ds5-41-S25 | Ds5-857-R25 |
| Precursor siRNA-3 | Ds3-8864-S25 | Ds3-9537-R25 |
| Precursor siRNA-4 | Ds3-8864-S25 | Ds3-9611-R23 |
| Precursor siRNA-5 | Ds5-41-S25 | Ds5-397-R23 |
| Precursor siRNA-6 | Ds3-9267-S23 | Ds3-9611-R23 |
| Precursor siRNA-7 | Ds5-201-S25 | Ds5-397-R23 |
| Precursor siRNA-8 | Ds5-261-S25 | Ds5-360-R25 |
| Precursor siRNA-9 | Ds5-311-S25 | Ds5-360-R25 |

**Table 2**

| Primer | Sequence |
|---|---|
| Ds5-41-S25 | ACTCCCCTGTGAGGAACTACTGTCT (SEQ ID No. 35) |
| Ds3-8864-S25 | AGGATGATTCTGATGACCCATTTCT (SEQ ID No. 36) |
| Ds3-9267-S23 | GCGGGGGAGACATATATCACAGC (SEQ ID No. 37) |
| Ds5-201-S25 | TGGATCAACCCGCTCAATGCCTGGA (SEQ ID No. 38) |
| Ds5-261-S25 | TAGTGTTGGGTCGCGAAAGGCCTTG (SEQ ID No. 39) |
| Ds5-311-S25 | GAGTGCCCCGGGAGGTCTCGTAGAC (SEQ ID No. 40) |
| Ds5-612-R23 | CCCTCGTTGCCATAGAGGGGCCA (SEQ ID No. 41) |
| Ds5-857-R25 | AACCGGGCAAATTCCCTGTTGCATA (SEQ ID No. 42) |
| Ds3-9537-R25 | GACTAGGGCTAAGATGGAGCCACCA (SEQ ID No. 43) |
| Ds3-9611-R23 | ACATGATCTGCAGAGAGGCCAGT (SEQ ID No. 44) |
| Ds5-397-R23 | GCGGCGGTTGGTGTTACGTTTGG (SEQ ID No. 45) |
| Ds5-360-R25 | TTAGGATTTGTGCTCATGATGCACG (SEQ ID No. 46) |

Then, Dicer siRNA Generation kit (Cat # T510001) available from Gene Therapy Systems. Inc. was used and 10 to 20 µg each of dsRNA was reacted with 10 unit (20 µl) of the dicer protein of the kit (reaction liquid volume: 100 µl; 16 to 20 hours). After the double-stranded RNA mixture of short strands (d-siRNA) cleaved by the dicer was confirmed in 3% agarose gel electrophoresis, desalination and removing of un-cleaved RNA were performed with the column attached to the kit, and finally d-siRNA of 22 bp was confirmed by agarose gel. Concentration was measured by absorption and it was adjusted tc 5 µM with sterilized water and stored at -80°C until it was used.

### Example 7: Transfection of siRNA (1)

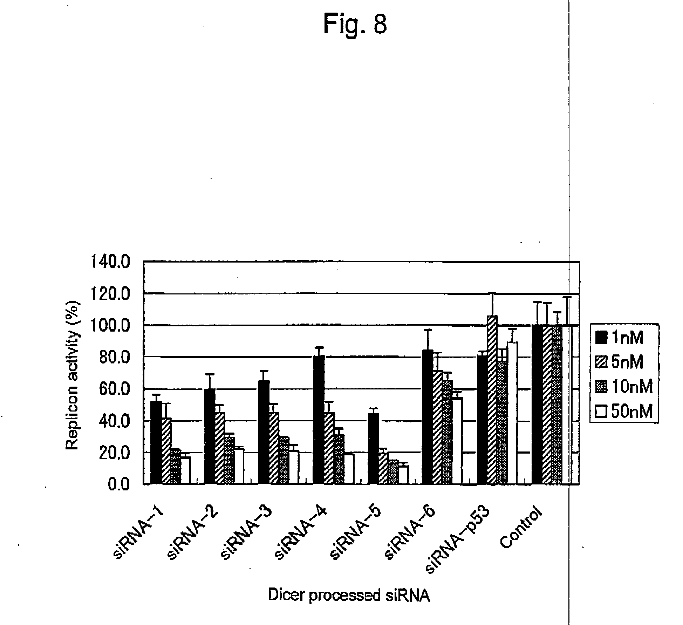

siRNAs prepared from double stranded precursor siRNA-1 to precursor siRNA-6 in Example 6 were introduced into the firefly luciferase HCV replicon cell (Huh-3-1) using the TransIT-TKO transfection reagent (Mirus Corporation cat. No. MIR2150) described in Example 4 in a concentration of 1 to 50 nM and luciferase activity was measured after 24 hours to determine the antivirotic activity. The results are shown in Figure 8. In the drawing, siRNA-p53 shows the result of the case in which siRNA by dicer processing of the oncogene p53 was added, and the control shows the result in which sterilized water was added.

Consequently, it has been demonstrated that siRNA prepared by dicer inhibited the replicative activity of HCV replicon in concentration dependence and has an antivirotic activity.

### Example 8: Transfection of siRNA (2)

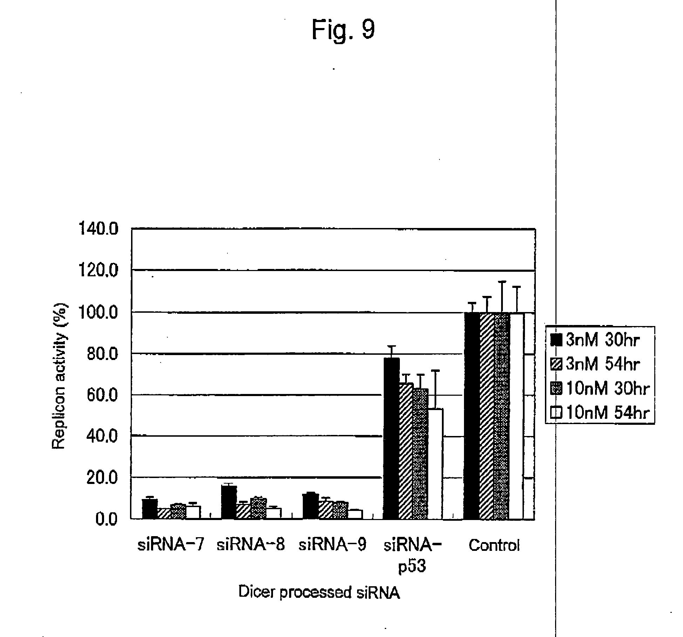

siRNAs prepared from double stranded precursor siRNA-7 to precursor siRNA-9 in Example 6 were introduced into the firefly luciferase HCV replicon cell (Huh-3-1) using the TransIT-TKO transfection reagent (Mirus Corporation cat. No. MIR2150) described in Example 4 in a concentration of 3 nM or 10 nM and luciferase activity was measured after 30 hours or 54 hours to determine the antivirotic activity. The results are shown in Figure9.

Consequently, it has been demonstrated that siRNA prepared by dicer inhibited the replicative activity of HCV replicon in concentration dependence and has an antivirotic activity as in Example 7.

### Industrial Applicability

As described in full detail above, the present invention has provided an oligoribonucleotide or a peptide nucleic acid which sequence-specifically binds to the HCV-RNA and inhibits activities of HCV, and a therapeutic agent for hepatitis C which contains these components as an active ingredient and enabled to provide a new and definite therapeutic method of HCV.

It should be noted that all the publications, patents and patent applications cited in this specification are entirely incorporated in this specifications as reference.

The invention also relates to the following aspects as defined in the following numbered paragraphs:
1. An oligoribonucleotide or peptide nucleic acid which sequence-specifically binds to the RNA of a hepatitis C virus (HCV).
2. The oligoribonucleotide or peptide nucleic acid according to paragraph 1 which hybridizes with the RNA of HCV under stringent conditions.
3. The oligoribonucleotide or peptide nucleic acid according to paragraph 1 **characterized in that** the oligoribonucleotide or peptide nucleic acid hybridizes with the sequence of a 5' non-coding region of the RNA of HCV.
4. The oligoribonucleotide or peptide nucleic acid according to paragraph 1 **characterized in that** the oligoribonucleotide or peptide nucleic acid hybridizes with the sequence of a highly identical region of the genetic sequences of a plurality of types of HCV different in genotype.
5. The oligoribonucleotide or peptide nucleic acid according to paragraph 1 which is a double-stranded RNA.
6. The oligoribonucleotide or peptide nucleic acid according to paragraph 1 which has a chain length of 19 to 23 bp.
7. An oligoribonucleotide having a nucleotide sequence shown in any one of SEQ ID Nos. 20 to 34.
8. An oligoribonucleotide which hybridizes under stringent conditions either with an RNA region of HCV having a sequence complementary to the oligoribonucleotide according to paragraph 7 or an RNA region of HCV hybridizing under stringent conditions with said oligoribonucleotide.
9. An oligoribonucleotide represented by a nucleotide sequence consisting of 19 to 23 contiguous bases in any one of the nucleotide sequences shown in SEQ ID Nos. 47 to 55.
10. An oligoribonucleotide which hybridizes under stringent condition either with an RNA region of HCV having a sequence complementary to the oligoribonucleotide according to paragraph 9 or an RNA region of HCV hybridizing under stringent conditions with said oligoribonucleotide.
11. A vector which expresses the oligoribonucleotide according to any one of paragraphs 1 to 10.
12. A therapeutic agent for hepatitis C containing as an active ingredient the oligoribonucleotide or peptide nucleic acid according to any one of paragraphs 1 to 10 or the vector according to paragraph 11.
13. A method of inhibiting replication ability of HCV by allowing the oligoribonucleotide or peptide nucleic acid according to any one of paragraphs 1 to 10 to bind to the HCV-RNA.

SEQUENCE LISTING

<110> TOKYO METROPOLITAN ORGANIZATION FOR MEDICAL RESEARCH
      CHUGAI SEIYAKU KABUSHIKI KAISHA

<120> Oligoribonucleotide or Peptidic Nucleic Acid Inhibiting Function of
Hepatitis C Virus

<130> P023642EPA

<150> JP 2003-016750
<151> 2003-01-24

<160> 56

<170> PatentIn Ver. 2.1

<210> 1
<211> 500
<212> DNA
<213> Hepatitis C virus

<400> 1
gccagccccc tgatgggggc gacactccac catgaatcac tcccctgtga ggaactactg 60
tcttcacgca gaaagcgtct agccatggcg ttagtatgag tgtcgtgcag cctccaggac 120
ccccccctccc gggagagcca tagtggtctg cggaaccggt gagtacaccg gaattgccag 180
gacgaccggg tcctttcttg gatcaacccg ctcaatgcct ggagatttgg gcgtgccccc 240
gcaagactgc tagccgagta gtgttgggtc gcgaaaggcc ttgtggtact gcctgatagg 300
gtgcttgcga gtgccccggg aggtctcgta gaccgtgcac catgagcacg aatcctaaac 360
ctcaaaaaaa aaacaaacgt aacaccaacc gtcgcccaca ggacgtcaag ttcccgggtg 420
gcggtcagat cgttggtgga gtttacttgt tgccgcgcag gggccctaga ttgggtgtgc 480
gcgcgacgag aaagacttcc 500


<210> 2
<211> 500
<212> DNA
<213> Hepatitis C virus

<400> 2
cgattggggg cgacactcca ccatagatca ctcccctgtg aggaactact gtcttcacgc 60
agaaagcgtc tagccatggc gttagtatga gtgtcgtgca gcctccagga ccccccctcc 120
cgggagagcc atagtggtct gcggaaccgg tgagtacacc ggaattgcca ggacgaccgg 180
gtcctttctt ggatcaaccc gctcaatgcc tggagatttg ggcgtgcccc cgcgagactg 240
ctagccgagt agtgttgggt cgcgaaaggc cttgtggtac tgcctgatag ggtgcttgcg 300
agtgccccgg gaggtctcgt agaccgtgca ccatgagcac gaatcctaaa cctcaaagaa 360
aaaccaaacg taacaccaac cgccgcccac aggacgtcaa gttcccgggc ggtggtcaga 420
tcgttggtgg agtttacctg ttgccgcgca ggggccccag gttgggtgtg cgcgcgccca 480
ggaagacttc cgagcggtcg 500


<210> 3
<211> 500
<212> DNA
<213> Hepatitis C virus

<400> 3
ttgggggcga cactccacca tagatcactc ccctgtgagg aactactgtc ttcacgcaga 60
aagcgtctag ccatggcgtt agtatgagtg ttgtgcagcc tccaggaccc cccctcccgg 120
gagagccata gtggtctgcg gaaccggtga gtacaccgga attgccagga cgaccgggtc 180
ctttcttgga tcaacccgct caatgcctgg agatttgggc gtgcccccgc gagactgcta 240
gccgagtagt gttgggtcgc gaaaggcctt gtggtactgc ctgatagggt gcttgcgagt 300
gccccgggag gtctcgtaga ccgtgcatca tgagcacaaa tcctaaacct caaagaaaaa 360
ccaaacgtaa caccaaccgc cgcccacagg acgttaagtt cccgggcggt ggtcagatcg 420

ttggtggagt ttacctgttg ccgcgcaggg gccccaggtt gggtgtgcgc gcgactagga 480
agacttccga gcggtcgcaa 500

<210> 4
<211> 500
<212> DNA
<213> Hepatitis C virus

<400> 4
gggccagccc ccgattgggg gcgacactcc accatagatc actcccctgt gaggaactac 60
tgtcttcacg cagaaagcgt ctagccatgg cgttagtatg agtgtcgtgc agcctccagg 120
accccccctc ccgggagagc catagtggtc tgcggaaccg gtgagtacac cggaattgcc 180
aggacgaccg ggtcctttct tggatcaacc cgctcaatgc ctggagattt gggcgtgccc 240
ccgcgagact gctagccgag tagtgttggg tcgcgaaagg ccttgtggta ctgcctgata 300
gggtgcttgc gagtgccccg ggaggtctcg tagaccgtgc atcatgagca caaatcccaa 360
accccaaaga aaaaccaaac gtaacaccaa ccgtcgccca caggacgtca agttcccggg 420
tggtggtcag atcgttggtg gagtttacct gttgccgcgc aggggcccca ggttgggtgt 480
gcgcgcgact aggaagactt 500

<210> 5
<211> 500
<212> DNA
<213> Hepatitis C virus

<400> 5
   acccgccccc taataggggc gacactccgc catgaatcac tcccctgtga ggaactactg 60
   tcttcacgca gaaagcgtct agccatggcg ttagtatgag tgtcgtacag cctccaggcc 120
   cccccctccc gggagagcca tagtggtctg cggaaccggt gagtacaccg gaattgccgg 180
   gaagaccggg tcctttcttg gataaacccg ctctatgccc ggccatttgg gcgtgccccc 240
   gcaagactgc tagccgagta gcgttgggtt gcgaaaggcc ttgtggtact gcctgatagg 300
   gtgcttgcga gtgccccggg aggtctcgta gaccgtgcac catgagcaca aatcctaaac 360
   ctcaaagaaa aacccaaaga aacactaacc gtcgcccaca agacgttaag tttccgggcg 420
   gcggccagat cgttggcgga gtatacttgt tgccgcgtag gggccccaga ttgggtgtgc 480
   gcacagcaag gaagacttcg 500

<210> 6
<211> 500
<212> DNA
<213> Hepatitis C virus

<400> 6
   acccgccccc taataggggc gacactccgc catgaatcac tcccctgtga ggaactactg 60
   tcttcacgca gaaagcgtct agccatggcg ttagtatgag tgtcgtacag cctccaggcc 120
   cccccctccc gggagagcca tagtggtctg cggaaccggt gagtacaccg gaattgccgg 180
   gaagactggg tcctttcttg gataaaccca ctctatgccc ggccatttgg gcgtgccccc 240
   gcaagactgc tagccgagta gcgttgggtt gcgaaaggcc ttgtggtact gcctgatagg 300
   gtgcttgcga gtgccccggg aggtctcgta gaccgtgcac catgagcaca aatcctaaac 360
   ctcaaagaaa aacccacaga aacactaacc gtcgcccaca agacgttaag tttccgggcg 420
   gcggccagat cgttggcgga gtatacttgt tgccgcgcag gggccctaga ttgggtgtgc 480
   gcacgacaag gaagacttcg 500

<210> 7
<211> 500
<212> DNA
<213> Hepatitis C virus

<400> 7
   acccgcccct aataggggcg acactccgcc atgaaccact cccctgtgag gaactactgt 60
   cttcacgcag aaagcgtcta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gtcatttggg cgtgcccccg 240
   caagactgct agccgagtag cgttgggttg cgaaaggcct tgtggtactg cctgataggg 300
   tgcttgcgag tgccccggga ggtctcgtag accgtgcacc atgagcacaa atcctaaacc 360
   tcaaagaaaa accaaaagaa acaccaaccg tcgcccacaa gacgttaagt ttccgggcgg 420
   cggccagatc gttggcggag tatacttgtt gccgcgcagg ggccccaggt tgggtgtgcg 480
   cgcgacaagg aagacttcgg 500

<210> 8
<211> 500
<212> DNA
<213> Hepatitis C virus

<400> 8
acctgcccct aataggggcg acactccgcc atgaatcact cccctgtgag gaactactgt 60
cttcacgcag aaagcgccta gccatggcgt tagtatgagt gtcgtacagc ctccaggccc 120
ccccctcccg ggagagccat agtggtctgc ggaaccggtg agtacaccgg aattgccggg 180
aagactgggt cctttcttgg ataaacccac tctatgcccg gccatttggg cgtgcccccg 240
caagactgct agccgagtag cgttgggttg cgaaaggcct tgtggtactg cctgataggg 300
cgcttgcgag tgccccggga ggtctcgtag accgtgcacc atgagcacaa atcctaaacc 360
tcaaagaaaa accaaaagaa acaccaaccg tcgcccagaa gacgttaagt tcccgggcgg 420
cggccagatc gttggcggag tatacttgtt gccgcgcagg ggccccaggt tgggtgtgcg 480
cacgacaagg aaaacttcgg 500

<210> 9
<211> 500
<212> DNA
<213> Hepatitis C virus

<400> 9
acccgccccc taataggggc gacactccgc catgaatcac tcccctgtga ggaactactg 60
tcttcacgca gaaagcgtct agccatggcg ttagtatgag tgtcgtacag cctccaggcc 120
cccccctccc gggagagcca tagtggtctg cggaaccggt gagtacaccg gaattgccgg 180
gaagactggg tcctttcttg gataaaccca ctctatgccc ggccatttgg gcgtgccccc 240
gcaagactgc tagccgagta gcgttgggtt gcgaaaggcc ttgtggtact gcctgatagg 300
gtgcttgcga gtgccccggg aggtctcgta gaccgtgcac catgagcaca aatcctaaac 360
ctcaaagaaa aacccacaga aacactaacc gtcgcccaca agacgttaag tttccgggcg 420
gcggccagat cgttggcgga gtatacttgt tgccgcgcag gggccctaga ttgggtgtgc 480
gcacgacaag gaagacttcg 500

<210> 10
<211> 500
<212> DNA
<213> Hepatitis C virus

<400> 10
acccgcccct aataggggcg acactccgcc atgaatcact cccctgtgag gaactactgt 60
cttcacgcag aaagcgtcta gccatggcgt tagtatgagt gtcgtacagc ctccaggccc 120
ccccctcccg ggagagccat agtggtctgc ggaaccggtg agtacaccgg aattgccggg 180
aagactgggt cctttcttgg ataaacccac tctatgcccg gccatttggg cgtgcccccg 240
caagaccgct agccgagtag cgttgggttg cgaaaggcct tgtggtactg cctgataggg 300
tgcttgcgag tgccccggga ggtctcgtag accgtgcacc atgagcacaa atcctaaacc 360
tcaaagacaa accaaaagaa acaccagccg tcgcccacaa gacgttaggt ttccgggcgg 420
cggccagatc gttggcggag tatacttgtt gccgcgcagg ggccccaggt tgggtgtgcg 480
cgcgacaagg aagacttcgg 500

<210> 11
<211> 500
<212> DNA
<213> Hepatitis C virus

<400> 11
gcccgccccc tgatgggggc gacactccgc catgaatcac tcccctgtga ggaactactg 60
tcttcacgca gaaagcgtct agccatggcg ttagtatgag tgtcgtacag cctccaggcc 120
ccccctcccc gggagagcca tagtggtctg cggaaccggt gagtacaccg gaattaccgg 180
aaagactggg tcctttcttg gataaaccca ctctatgtcc ggtcatttgg gcacgccccc 240
gcaagactgc tagccgagta gcgttgggtt gcgaaaggcc ttgtggtact gcctgatagg 300
gtgcttgcga gtgccccggg aggtctcgta gaccgtgcat catgagcaca aatcctaaac 360
ctcaaagaaa aaccaaaaga aacacaaacc gccgcccaca ggacgttaag ttcccgggtg 420
gcggtcagat cgttggcgga gtttacttgc tgccgcgcag gggccccagg ttgggtgtgc 480
gcgcgacaag gaagacttct 500

<210> 12
<211> 311
<212> DNA
<213> Hepatitis C virus

<400> 12
   gcgtgtctca tgcccggccc cgctggttct ggttttgcct actcctgctc gctgcagggg 60
   taggcatcta cctcctcccc aacccatgaa ggttggggta aacactccgg cctcttaagc 120
   catttcctgt tttttttttt tttttttttt tttttttctt tttttttttc tttcctttcc 180
   ttcttttttt cctttctttt tcccttcttt aatggtggct ccatcttagc cctagtcacg 240
   gctagctgtg aaaggtccgt gagccgcatg actgcagaga gtgctgatac tggcctctct 300
   gcagatcatg t 311

<210> 13
<211> 371
<212> DNA
<213> Hepatitis C virus

<400> 13
gtccagctgg ttcgtggctg gttacagcgg gggagacata tatcacagcc tgtctcgtgc 60
ccgaccccgc tggttcatgt tgtgcctact cctactttca gtaggggtag gcatctacct 120
gctccccaac cgataaacgg ggagctaaac actccaggcc aataggccat ttcttttttt 180
tttttttttt tttttctttt tttttttttt tttttttttt tttttttttt tttttttttt 240
ctttcttttg tttttttttt ttttcttctt tttggtggct ccatcttagc cctagtcacg 300
gctagctgtg aaaggtccgt gagccgcatg actgcagaga gtgctgatac tggcctctct 360
gcagatcatg t 371

<210> 14
<211> 439
<212> DNA
<213> Hepatitis C virus

<400> 14
tgggcggtga agaccaagct caaactcact ccattgccgg aagcgcgcct cctggattta 60
tccagctggt tcactgtcgg cgccggcggg ggcgacattt atcacagcgt gccgcgtgcc 120
cgaccccgct tattactcct tggcctactc ctactttttg taggggtagg ccttttccta 180
ctccccgctc ggtagagcgg cacacattag ctacactcca tagctaactg tccctttttt 240
tttgtttttt tttttttttt tttttttttt ttttcttttt tttttttttt tttgtttctt 300
ttccttctca tttccttctt atcttaatta cttcctttcc tggtggctcc atcttagccc 360
tagtcacggc tagctgtgaa aggtccgtga gccgcatgac tgcagagatt gccgtaactg 420
gtatctctgc agatcatgt 439

<210> 15
<211> 347
<212> DNA
<213> Hepatitis C virus

<400> 15
cctggattta tccagctggt tcactgtcgg cgccggcggg ggcgacattt atcacagcgt 60

gccgcgtgcc cgaccccgct tattactcct tggcctactc ctactttttg taggggtagg 120
ccttttccta ctccccgctc ggtagagcgg cacacattag ctacactcca tagctaactg 180
tccctttttt tttttttttt tgtttctttt ccttctcatt tccttcttat cttaattact 240
ttctttcctg gtggctccat cttagcccta gtcacggcta gctgtgaaag gtccgtgagc 300
cgcatgactg cagagattgc cgtaactggt atctctgcag atcatgt 347

<210> 16
<211> 360
<212> DNA
<213> Hepatitis C virus

<400> 16
   tttatccagt tggtttaccg tcggcgccgg cgggggcgac atttatcaca gcgtgtcgcg 60
   tgcccgaccc cgcttattac tccttagcct actcctactt ttcgtagggg taggcctctt 120
   tttactcccc gctcggtaga gcggcacaca ttagctacac tccatagcta actgttcctt 180
   tttttttttt tttttttttt tttttttttt tttttttttt tttttttttt tttttttttt 240
   ttttttttct ttccttcctt tctcaccttc ttttacttct ttcctggtgg ctccatctta 300
   gccctagtca cggctagctg tgaaaggtcc gtgagccgca tgactgcaga gagtgccgta 360

<210> 17
<211> 378
<212> DNA
<213> Hepatitis C virus

<400> 17
ggacttatcc agttggttca ccgtcggcgc cggcgggggc gacatttttc acagcgtgtc 60
gcgcgcccga ccccgctcat tactcttcgg cctactccta cttttcgtag gggtaggcct 120
cttcctactc cccgctcggt agagcggcac acactaggta cactccatag ctaactgttc 180
cttttttttt tttttttttt tttttttttt tttttttttt ttttcttttt tttttttttc 240
cctctttctt cccttctcat cttattctac tttctttctt ggtggctcca tcttagccct 300
agtcacggct agctgtgaaa ggtccgtgag ccgcatgact gcagagagtg ccgtaactgg 360
tctctctgca gatcatgt 378

<210> 18
<211> 374
<212> DNA
<213> Hepatitis C virus

<400> 18
ggatttgtcc agttggttta ccgtcggcgc cggcgggggc gacatttatc acagcgtgtc 60
gcgtgcccga ccccgcctat tactccttag cctactccta ctttctgtag gggtaggcct 120
cttcctactc cccgctcgat agagcggcac acattagcta cactccatag ctaactgttc 180
cttttttttt tttttttttt tttttttttt tttttttttc tttttttttt tttttccctc 240
tttcttccct tctcatctta ttctactttc tttcttggtg gctccatctt agccctggtc 300
acggctagct gtgaaaggtc cgtgagccgc atgactgcag agagtgccgt aactggtctc 360
tctgcagatc atgt 374

<210> 19
<211> 354
<212> DNA
<213> Hepatitis C virus

<400> 19
tagatttatc cgggtggttc accgtgggcg ccggcggggg cgacatcttt cacagcgtgt 60
cgcatgcccg accccgccta ttactccttt gcctactcct acttagcgta ggagtaggca 120
tctttttact ccccgctcgg tagagcggca aaccctagct acactccata gctagttttc 180
tttttttttt tttttttttt ttttgttttt tttttttttc ctctttttcc gtattttttt 240
tttttcctct tttcttggtg gctccatctt agccctagtc acggctagct gtgaaaggtc 300
cgtgagccgc atgactgcag agagtgccgt aactggtctc tctgcagatc atgt 354

<210> 20
<211> 21
<212> RNA
<213> Artificial Sequence

<220>
<223> 5'-UTR target siRNA

<400> 20
ggaacuacug ucuucacgca g                                                21


<210> 21
<211> 21
<212> RNA
<213> Artificial Sequence

<220>
<223> 5'-UTR target siRNA

<400> 21
gccauagugg ucugcggaac c                                                21


<210> 22
<211> 22
<212> RNA
<213> Artificial Sequence

<220>
<223> 5'-UTR target siRNA

<400> 22
aggccuugug guacugccug au                                               22


<210> 23
<211> 20
<212> RNA
<213> Artificial Sequence

<220>
<223> 5'-UTR target siRNA

<400> 23
gucucguaga ccgugcauca                                                  20


<210> 24
<211> 21
<212> DNA
<213> Artificial Sequence

<220>
<223> 5'-UTR target siRNA

<400> 24
gcgaaaggcc ttgtggtact g                                                21


<210> 25
<211> 20
<212> DNA

<213> Artificial Sequence

<220>
<223> 5'-UTR target siRNA

<400> 25
gtctcgtaga ccgtgcacca                                                    20


<210> 26
<211> 21
<212> DNA
<213> Artificial Sequence

<220>
<223> 5'-UTR target siRNA

<400> 26
gucucguaga ccgugcauca t                                                  21


<210> 27
<211> 21
<212> RNA
<213> Artificial Sequence

<220>
<223> 5'-UTR target siRNA

<400> 27
ggaacuacug ucuucacgca g                                                  21


<210> 28
<211> 21
<212> RNA
<213> Artificial Sequence

<220>
<223> 5'-UTR target siRNA

<400> 28
gccauagugg ucugcggaac c                                                  21


<210> 29
<211> 22
<212> RNA
<213> Artificial Sequence

<220>
<223> 5'-UTR target siRNA

<400> 29
aggccuugug guacugccug au                                                 22


<210> 30
<211> 20
<212> RNA
<213> Artificial Sequence

<220>
<223> 5'-UTR target siRNA

<400> 30
gucucguaga ccgugcauca                                                    20

<210> 31
<211> 21
<212> DNA
<213> Artificial Sequence

<220>
<223> 5'-UTR target siRNA

<400> 31
gcgaaaggcc ttgtggtact g                                                  21

<210> 32
<211> 20
<212> DNA
<213> Artificial Sequence

<220>
<223> 5'-UTR target siRNA

<400> 32
gtctcgtaga ccgtgcacca                                                    20

<210> 33
<211> 21
<212> DNA
<213> Artificial Sequence

<220>
<223> 3'-UTR target siRNA

<400> 33
ggctccatct tagccctagt c                                                  21

<210> 34
<211> 21
<212> DNA
<213> Artificial Sequence

<220>
<223> 3'-UTR target siRNA

<400> 34
ggctagctgt gaaaggtccg t                                                  21

<210> 35
<211> 25
<212> DNA
<213> Artificial Sequence

<220>
<223> primer Ds5-41-S25

<400> 35
actcccctgt gaggaactac tgtct                                              25

<210> 36
<211> 25
<212> DNA
<213> Artificial Sequence

<220>
<223> primer Ds3-8864-S25

<400> 36
aggatgattc tgatgaccca tttct                                          25


<210> 37
<211> 23
<212> DNA
<213> Artificial Sequence

<220>
<223> primer Ds3-9267-S23

<400> 37
gcgggggaga catatatcac agc                                            23


<210> 38
<211> 25
<212> DNA
<213> Artificial Sequence

<220>
<223> primer Ds5-201-S25

<400> 38
tggatcaacc cgctcaatgc ctgga                                          25


<210> 39
<211> 25
<212> DNA
<213> Artificial Sequence

<220>
<223> primer Ds5-261-S25

<400> 39
tagtgttggg tcgcgaaagg ccttg                                          25


<210> 40
<211> 25
<212> DNA
<213> Artificial Sequence

<220>
<223> primer Ds5-311-S25

<400> 40
gagtgccccg ggaggtctcg tagac                                          25


<210> 41
<211> 23
<212> DNA

<213> Artificial Sequence

<220>
<223> primer Ds5-612-R23

<400> 41
ccctcgttgc catagagggg cca                                              23


<210> 42
<211> 25
<212> DNA
<213> Artificial Sequence

<220>
<223> primer Ds5-857-R25

<400> 42
aaccgggcaa attccctgtt gcata                                            25


<210> 43
<211> 25
<212> DNA
<213> Artificial Sequence

<220>
<223> primer Ds3-9537-R25

<400> 43
gactagggct aagatggagc cacca                                            25


<210> 44
<211> 23
<212> DNA
<213> Artificial Sequence

<220>
<223> primer Ds3-9611-R23

<400> 44
acatgatctg cagagaggcc agt                                              23


<210> 45
<211> 23
<212> DNA
<213> Artificial Sequence

<220>
<223> primer Ds5-397-R23

<400> 45
gcggcggttg gtgttacgtt tgg                                              23


<210> 46
<211> 25
<212> DNA
<213> Artificial Sequence

<220>
<223> primer Ds5-360-R25

<400> 46
ttaggatttg tgctcatgat gcacg                                          25

<210> 47
<211> 572
<212> DNA
<213> Artificial Sequence

<220>
<223> PCR product siRNA-1

<400> 47
actcccctgt gaggaactac tgtcttcacg cagaaagcgt ctagccatgg cgttagtatg 60
agtgtcgtgc agcctccagg accccccctc ccgggagagc catagtggtc tgcggaaccg 120
gtgagtacac cggaattgcc aggacgaccg ggtcctttct tggatcaacc cgctcaatgc 180
ctggagattt gggcgtgccc ccgcgagact gctagccgag tagtgttggg tcgcgaaagg 240
ccttgtggta ctgcctgata gggtgcttgc gagtgccccg ggaggtctcg tagaccgtgc 300
atcatgagca caaatcctaa accccaaaga aaaaccaaac gtaacaccaa ccgccgccca 360
caggacgtca agttcccggg tggtggtcag atcgttggtg gagtttacct gttgccgcgc 420
aggggcccca ggttgggtgt gcgcgcgact aggaagactt ccgagcggtc acaacctcgt 480
ggaaggcgac aacctatccc caaggctcgc cagcccgagg gcagggcctg ggctcagccc 540
gggtaccctt ggcccctcta tggcaacgag gg                                  572

<210> 48
<211> 817
<212> DNA
<213> Artificial Sequence

<220>
<223> PCR product siRNA-2

<400> 48
actcccctgt gaggaactac tgtcttcacg cagaaagcgt ctagccatgg cgttagtatg 60
agtgtcgtgc agcctccagg accccccctc ccgggagagc catagtggtc tgcggaaccg 120
gtgagtacac cggaattgcc aggacgaccg ggtcctttct tggatcaacc cgctcaatgc 180
ctggagattt gggcgtgccc ccgcgagact gctagccgag tagtgttggg tcgcgaaagg 240
ccttgtggta ctgcctgata gggtgcttgc gagtgccccg ggaggtctcg tagaccgtgc 300
atcatgagca caaatcctaa accccaaaga aaaaccaaac gtaacaccaa ccgccgccca 360
caggacgtca agttcccggg tggtggtcag atcgttggtg gagtttacct gttgccgcgc 420
aggggcccca ggttgggtgt gcgcgcgact aggaagactt ccgagcggtc acaacctcgt 480
ggaaggcgac aacctatccc caaggctcgc cagcccgagg gcagggcctg ggctcagccc 540
gggtaccctt ggcccctcta tggcaacgag ggcatggggt gggcaggatg gctcctgtca 600
ccccgcggct cccggcctag ttggggcccc acggaccccc ggcgtaggtc gcgtaatttg 660
ggtaaggtca tcgataccct cacatgcggc ttcgccgacc tcatggggta cattccgctc 720
gtcggcgccc ccctaggggg cgttgccagg gccctggcac atggtgtccg ggttgtggag 780
gacggcgtga actatgcaac agggaatttg cccggtt                             817

<210> 49
<211> 674
<212> DNA
<213> Artificial Sequence

<220>
<223> PCR product siRNA-3

<400> 49
aggatgattc tgatgaccca tttcttctcc atccttctag cccaggagca acttgaaaaa 60
gccctggatt gccagatcta cggggcctgt tactccattg agccacttga cctacctcag 120
atcattgaac gactccatgg tcttagcgca ttttcactcc atagttactc tccaggtgag 180
atcaataggg tggcttcatg cctcaggaaa cttggggtac cacccttgcg agtctggaga 240

catcgggcca gaagtgtccg cgctaagctg ctgtcccagg gggggagggc tgccacttgt 300
ggtaagtacc tcttcaactg ggcagtaagg accaagctca aactcactcc aatcccggca 360
gcgtcccagt tggacttgtc cagctggttc gtggctggtt acagcggggg agacatatat 420
cacagcctgt ctcgtgcccg accccgctgg ttcatgttgt gcctactcct actttcagta 480
ggggtaggca tctacctgct ccccaaccga taaacgggga gctaaacact ccaggccaat 540
aggccatttc tttttttttt tttttttttt tttctttttt tttttttttt tttttttttt 600
tttttttttt tttttttctt tcttttgttt tttttttttt tcttcttttt ggtggctcca 660
tcttagccct agtc 674

<210> 50
<211> 748
<212> DNA
<213> Artificial Sequence

<220>
<223> PCR product siRNA-4

<400> 50
aggatgattc tgatgaccca tttcttctcc atccttctag cccaggagca acttgaaaaa 60
gccctggatt gccagatcta cggggcctgt tactccattg agccacttga cctacctcag 120
atcattgaac gactccatgg tcttagcgca ttttcactcc atagttactc tccaggtgag 180
atcaataggg tggcttcatg cctcaggaaa cttggggtac cacccttgcg agtctggaga 240
catcgggcca gaagtgtccg cgctaagctg ctgtcccagg gggggagggc tgccacttgt 300
ggtaagtacc tcttcaactg ggcagtaagg accaagctca aactcactcc aatcccggca 360
gcgtcccagt tggacttgtc cagctggttc gtggctggtt acagcggggg agacatatat 420
cacagcctgt ctcgtgcccg accccgctgg ttcatgttgt gcctactcct actttcagta 480
ggggtaggca tctacctgct ccccaaccga taaacgggga gctaaacact ccaggccaat 540
aggccatttc tttttttttt tttttttttt tttctttttt tttttttttt tttttttttt 600
tttttttttt tttttttctt tcttttgttt tttttttttt tcttcttttt ggtggctcca 660
tcttagccct agtcacggct agctgtgaaa ggtccgtgag ccgcatgact gcagagagtg 720
ctgatactgg cctctctgca gatcatgt 748

<210> 51
<211> 357
<212> DNA
<213> Artificial Sequence

<220>
<223> PCR product siRNA-5

<400> 51
actcccctgt gaggaactac tgtcttcacg cagaaagcgt ctagccatgg cgttagtatg 60
agtgtcgtgc agcctccagg accccccctc ccgggagagc catagtggtc tgcggaaccg 120
gtgagtacac cggaattgcc aggacgaccg ggtcctttct tggatcaacc cgctcaatgc 180
ctggagattt gggcgtgccc ccgcgagact gctagccgag tagtgttggg tcgcgaaagg 240
ccttgtggta ctgcctgata gggtgcttgc gagtgccccg ggaggtctcg tagaccgtgc 300
atcatgagca caaatcctaa accccaaaga aaaaccaaac gtaacaccaa ccgccgc 357

<210> 52
<211> 345
<212> DNA
<213> Artificial Sequence

<220>
<223> PCR product siRNA-6

<400> 52
gcgggggaga catatatcac agcctgtctc gtgcccgacc ccgctggttc atgttgtgcc 60
tactcctact ttcagtaggg gtagg[?]atct acctgctccc caaccgataa acggggagct 120
aaacactcca ggccaatagg ccatttcttt tttttttttt tttttttttt cttttttttt 180
tttttttttt tttttttttt tttttttttt ttttctttct tttgtttttt tttttttct 240

tcttttggt ggctccatct tagccctagt cacggctagc tgtgaaaggt ccgtgagccg 300
catgactgca gagagtgctg atactggcct ctctgcagat catgt 345

<210> 53
<211> 197
<212> DNA
<213> Artificial Sequence

<220>
<223> PCR product siRNA-7

<400> 53
tggatcaacc cgctcaatgc ctggagattt gggcgtgccc ccgcgagact gctagccgag 60
tagtgttggg tcgcgaaagg ccttgtggta ctgcctgata gggtgcttgc gagtgccccg 120
ggaggtctcg tagaccgtgc atcatgagca caaatcctaa accccaaaga aaaaccaaac 180
gtaacaccaa ccgccgc 197

<210> 54
<211> 100
<212> DNA
<213> Artificial Sequence

<220>
<223> PCR product siRNA-8

<400> 54
tagtgttggg tcgcgaaagg ccttgtggta ctgcctgata gggtgcttgc gagtgccccg 60
ggaggtctcg tagaccgtgc atcatgagca caaatcctaa 100

<210> 55
<211> 50
<212> DNA
<213> Artificial Sequence

<220>
<223> PCR product siRNA-9

<400> 55
   gagtgccccg ggaggtctcg tagaccgtgc atcatgagca caaatcctaa 50

<210> 56
<211> 9611
<212> DNA
<213> Hepatitis C virus

<400> 56
gggccagccc ccgattgggg gcgacactcc accatagatc actcccctgt gaggaactac 60
tgtcttcacg cagaaagcgt ctagc[?]atgg cgttagtatg agtgtcgtgc agcctccagg 120
accccccctc ccgggagagc catagtggtc tgcggaaccg gtgagtacac cggaattgcc 180
aggacgaccg ggtcctttct tggat[?]aacc cgctcaatgc ctggagattt gggcgtgccc 240
ccgcgagact gctagccgag tagtgttggg tcgcgaaagg ccttgtggta ctgcctgata 300
gggtgcttgc gagtgccccg ggaggtctcg tagaccgtgc atcatgagca caaatcctaa 360
accccaaaga aaaaccaaac gtaacaccaa ccgccgccca caggacgtca agttcccggg 420
tggtggtcag atcgttggtg gagtttacct gttgccgcgc aggggcccca ggttgggtgt 480
gcgcgcgact aggaagactt ccgag[?]ggtc acaacctcgt ggaaggcgac aacctatccc 540
caaggctcgc cagcccgagg gcagg[?]cctg ggctcagccc gggtaccctt ggcccctcta 600
tggcaacgag ggcatggggt gggca[?]gatg gctcctgtca ccccgcggct cccggcctag 660
ttggggcccc acggaccccc ggcgtaggtc gcgtaatttg ggtaaggtca tcgataccct 720
cacatgcggc ttcgccgacc tcatg[?]ggta cattccgctc gtcggcgccc ccctaggggg 780
cgttgccagg gccctggcac atggt[?]tccg ggttgtggag gacggcgtga actatgcaac 840

agggaatttg cccggttgct ctttctctat cttcctcttg gctctgctgt cctgtttgac 900
catcccagct tccgcttatg aggtgcgcaa cgtatccggg atataccatg tcacgaacga 960
ctgctccaac tcaagtattg tgtatgaggc agcggacatg atcatgcata cccccgggtg 1020
cgtgccctgc gttcgggagg gcaactcctc ccgttgctgg gtggcactta ctcccacgct 1080
agcggccagg aatgccagcg tccccactac ggcaatacga cgccatgtcg atttgctcgt 1140
tggggcggct gctttctgct ccgctatgta tgtgggagat ctctgcggat ctgttttcct 1200
tgtctcccag ctgttcacct tctcgccccg ccggcatgag acaatacagg actgcaattg 1260
ctcaatctat cccggccacg tgtcaggtca ccgcatggct tgggacatga tgatgaactg 1320
gtcgcctaca acggccctgg tggtgtcgca gttactccgg atcccacaag ctatcgtgga 1380
catggtggcg ggggctcact ggggtgtcct agcgggcctt gcctactatt ccatggtggg 1440
gaactgggct aaggtattga ttgtgatgct actttttgcc ggcgtcgacg gggagacccg 1500
tgtgacaggg gggcagatag ccagaaatgc ctactcgctc acgaccctct tttcatctgg 1560
gtcggctcag aacatccagc tcataaacac caacggtagc tggcacatca acaggactgc 1620
cctgaactgc aatgactccc tcaacaccgg gtttcttgcc gcgctgttct acacgcacaa 1680
gttcaacgcg tccggatgtc cagagcgctt ggccagctgc cgccccattg acaagttcga 1740
tcaggggtgg ggtcccatca cttatgctga gcagggcggc caggaccaga ggccttattg 1800
ctggcactac gcacctaaac catgtggtat tgtatccgcg tcgaaggtgt gtggtccagt 1860
gtattgtttc accccaagcc cagttgtagt ggggacgacc gatcggttcg gtgtccctac 1920
gtatagctgg ggggagaatg agacagacgt gctgctcctt aacaacacgc ggccgccgca 1980
aggcaactgg ttcggctgta cgtggatgaa cggcactggg ttcaccaaga catgcggggg 2040
ccccccgtgt aacatcgggg ggggcggcaa taacaccttg acctgcccta cggactgttt 2100
ccggaagcac cccgcggcca cttacacaaa atgtggttcg ggaccttggc tgacacccag 2160
gtgcttggta gactacccat acaggctctg gcactacccc tgcactgcca actttaccat 2220
cttcaaggtt aggatgtatg taggggggcgt ggagcacagg ctcgatgctg catgcaattg 2280
gacccgaggg gaacgttgca acttggagga tagggataga ttggagctca gcccgctact 2340
gctgtctaca acagagtggc aggtgctgcc ctgttctttc accaccctac cggctctgtc 2400
cactggttta attcatctcc atcagaacat cgtggacgtg caatacctgt acggtatagg 2460
gtcggcagtt gtttcctttg caatcaaatg ggactatatc gtgatacttt tcctcctcct 2520
ggcggacgcg cgcgtctgtg cctgcttgtg gatgatgctg ctgatagccc aggccgaggc 2580
cgccttagaa aacctggtgg tcctcaatgc ggcgtccgtg gccggagcgc atggcattct 2640
ctccttcctt gtgttcttct gtgccgcctg gtacatcaag ggcaagctgg tccccggggc 2700
agcatatgct ttctatggag tatggccgct gctcctgctt ctgctggcct taccaccacg 2760
agcttacgct atggagcggg agatggctgc atcgtgcgga ggcgcggtgt ttgtaggtct 2820
ggtactcttg actttgtcac catactataa agagttcctc gccaggctca tatggtggtt 2880
gcaatatttt atcaccagag ccgaggcgca cctgcaagtg tggatccccc ccctcaacat 2940
tcgggggggc cgcgatgcca tcatcctcct cgcgtgtgta gtccacccag agctaatctt 3000
tgacatcacc aaactcctgc tcgccatact cggtccgctc atggtgctcc aggctagcat 3060
aactcaagtg ccgtacttcg tacgcgccca agggctcatt cgtgcatgca tgttggtgcg 3120
gaaggtagcc gggggccatt atgtccaaat ggcctttgtg aagctgaccg cactgacagg 3180
tacgtacgtt tatgaccatc taactccact gcgggactgg gcccacgcgg gcctgcgaga 3240
cctcgcggtg gcagtagagc ccgttgtctt ctctgacatg gagaccaagg tcatcacctg 3300
gggggcagac accgcagcgt gtggggacat tatcttgggt ctacctgtct ccgcccgaag 3360
gggtagggag atacttctgg ggccggccga tagtcttgaa gggcaggggt ggcggctcct 3420
tgctcccatc acggcctatt cccaacagac gcggggccta cttggttgca tcatcactag 3480
cctcacaggc cgggacaaaa accaagtcga gggggaggtt caagtggtct ccaccgcgac 3540
acaatccttc ctggcgacct gcgtcaatgg cgcgtgctgg actgtcttcc atggtgccgg 3600
ctcaaagacc ttagctggcc caaaaggtcc aatcacccag atgtacacta atgtagacct 3660
ggacctcgtc ggctggcagg cgccccccgg gtcgcgttct ctgacaccat gcacctgcgg 3720
cagctcagac ctctatttgg tcacgagaca tgctgatgtc attccggtgc gccggcgggg 3780
cgacagtagg ggaagcctac tctctcccag acctgtctcc tacttgaaag gctcctcggg 3840
tggtccgctg ctctgccctt cgaggcacgc tgtgggcatc ttccgggctg ctgtgtgcac 3900
ccggggggtt gcgaaggcgg tggatttcat acccgttgaa tcaatggaaa ctactatgcg 3960
gtctccggtc ttcacggata actcatcccc cccggccgta ccgcagacat tccaagtggc 4020
ccatctacac gcccctactg gcagcggcaa gagcactaag gtgccggctg catatgcagc 4080
ccaagggtat aaggtgctcg tcctgaaccc gtccgttgcc gctaccttgg gttttggggc 4140
gtatatgtct aaggcacatg gtatcgaccc caacatcaga actggggtaa gggccatcac 4200
cacgggcgcc cctattacat actccaccta cggcaagttc cttgccgacg gcggttgttc 4260
cggggcgcc tatgacatca taatatgtga tgagtgccac tcaactgact cgactaccat 4320
cttgggcatt ggcacagtcc tggaccaagc ggagacggct ggagcgcggc tcgtcgtgct 4380
cgccaccgct acgcctccgg gatcggtcac cgtgccacac cccaatattg aggaggtggc 4440
cctgtccaac gctggagaaa tcccctteta cggcaaagcc atccccattg aggtcatcaa 4500
gggggaaga catctcattt tctgccattc caagaagaag tatgacgagc tcgccgcaaa 4560
gctatcagcc ctcggactta atgctgtagc atattatcgg ggtcttgatg tgtccgtcat 4620
accgaccaac ggagacgtcg ttgtcgtggc aacagacgct ctaatgacgg gctttaccgg 4680

cgactttgac tcagtgatcg actgtaacac atgtgtcacc cagacagtcg atttcagcct 4740
ggatcccacc ttcaccatcg agacgacgac cgtgccccaa gacgcagtgg cgcgatcaca 4800
gcggcggggt aggactggta ggggcaggag aggcatctac aggtttgtga ctccaggaga 4860
acggccctcg ggcatgttcg attcctcggt cctgtgtgag tgctatgacg cgggctgtgc 4920
ttggtacgag ctcacgcctg ctgagacctc ggttaggttg cgggcttacc tgaatacacc 4980
agggttgccc gtctgccagg accatctgga gttttgggag agcgtctcca caggcctcac 5040
ccacatagat gcccatttc tgtcccagac taaacaggca ggagacaact tcccctacct 5100
ggtagcatac caagccacag tgtgcgccag agctcaagct ccacctccat catgggatca 5160
aatgtggaag tgtctcatac ggctcaaacc cacgctgcac gggccaacac ccctgctgta 5220
taggctagga gccgtccaaa atgagatcac cctcacacac cccatgacca aattcatcat 5280
ggcatgcatg tcggctgacc tggaggtcgt cactagcacc tgggtgctag taggcggagt 5340
ccttgcagct ctggctgcat attgcttgac aacaggcagt gtggtcattg tgggtaggat 5400
catcttgtcc gggaggccgg ctgttattcc cgacagggaa gtcctctacc gggagttcga 5460
tgagatggaa gagtgcgcct cacacctccc ttacatcgaa cagggaatgc agcttgccga 5520
gcaattcaag cagaaggcgc tcggattgct gcaaacagcc accaagcaag cggaggctgc 5580
tgctcccgtg gtagaatcca agtggcgagc ccttgagacc ttctgggcga agcacatgtg 5640
gaatttcatc agcgggatac agtacctagc aggcttgtcc actctgcctg ggaaccccgc 5700
gatagcatca ctgatggcat tcacagcctc tatcaccagc ccgctctcca cccagaatac 5760
cctattattt aacatctggg ggggatgggt ggctgcccaa ctcgcccccc ccagtgctgc 5820
ttcggctttc gtgggcgccg gtatcgccgg tgcggctgtc ggcagcatag gtcttgggaa 5880
ggtgcttgtg gacatcttgg cgggatatgg ggcaggggtg gctggcgcgc tcgtagcttt 5940
taagatcatg agcggcgagg tgccctccac cgaggacctg gttaacttac tccctgccat 6000
cctctctccc ggcgccctag tcgtcggggt cgtgtgcgca gcaatactgc gtcggcacgt 6060
gggcccggga gagggggctg tacagtggat gaaccggctg atagcgttcg cctcgcgggg 6120
taaccacgtt tcccccgcgc actatgtgcc tgagagcgac gctgcggcgc gtgttactca 6180
gatcctctcc ggccttacca tcactcagct gctgaagagg cttcaccact ggatcaatga 6240
ggactgctcc acgccatgct ccggttcgtg gctaagggat gtttgggact ggatatgcac 6300
ggtgttgact gacttcaaga cctggctcca gtccaagctc ctgccgcggt taccgggggt 6360
ccctttcttc tcgtgtcaac gcgggtacaa gggagtctgg cgggggacg gtatcatgca 6420
gaccacctgc ccgtgtggag cacagatcac cggacatgtc aaaaacggtt ccatgaggat 6480
cgtcgggcct aaaacctgca gcagcacgtg gcatggaacg ttccccatca acgcatacac 6540
cacaggccca tgcgcaccct ccccggcgcc aaactattcc agggcgctat ggcgggtggc 6600
cgctgaggag tacgtggagg ttacgcgggt gggggatttc cactacgtga cgggcatgac 6660
cactgacaac gtaaagtgcc catgccaggt tccggcccct gaattcttca ctgaggtgga 6720
tggagtgcgg ttgcacaggt acgctccggc gtgcaaaccc ctcctacggg aggaggtcac 6780
attccaggtt gggctcaacc aatacctggt tgggtcacag ctcccatgcg agcccgaacc 6840
ggatgtagca gtgctaactt ccatgcttac cgacccctcc cacatcacag cagagacggc 6900
aaagcgtagg ctggctaggg ggtctccccc ctccttggcc agttcttcag ctagccagtt 6960
atctgcgcct tccttgaagg cgacatgcac tacccatcat gactccccgg acgttgacct 7020
catcgaggcc aacctcctgt ggcggcagga gatgggcggg aacatcaccc gcgtggagtc 7080
agagaataag gtagtaattt tggactcttt cgatccgctc cgagcggagg aggacgagag 7140
ggaaccatcc gttgcggcgg agatcttgcg gaaaaccaag aggttccccc cggcgatgcc 7200
catatgggca cgcccggatt acaaccctcc gttgctagag tcctggaaag acccggacta 7260
cgtccctccg gtggtacacg ggtgcccgct accacctacc aaagctcctc cgataccacc 7320
cccacggaga aagaggacgg tagtcctgac agagtccact gtgtcttctg ccttggcgga 7380
gcttgctact aagacctttg gcagctccgg gtcgtcggcc gtcgacagcg gcacggcaac 7440
tgctcctccc gaccaggctt ccgacgacgg cgaccaagga tctgacgttg agtcgtattc 7500
ctccatgccc cctcttgagg gagagccggg ggaccccgat ctcagcgacg ggtcttggtc 7560
taccgtgagc gaggaggccg gtgaggacgt catctgctgc tcaatgtcct acacatggac 7620
aggcgccttg atcacgccat gcgccgcgga ggaaagcaag ttgcccatca acccgttgag 7680
caactctttg ttgcgtcacc acaacatggt ctatgctaca acatcccgca gcgcaggcct 7740
acggcagaag aaggtcacct ttgacagact gcaagtcctg gacgaccact accgggacgt 7800
gctcaaggag atgaaggcga aggcgtccac agttaaggct aaactcctat ccatagaaga 7860
agcctgtaag ctgacgcccc cacattcggc cagatccaaa tttggctatg gggcaaagga 7920
cgtccggaac ctatccagca aggccgttaa ccacatccgc tccgtgtgga aggacttgct 7980
ggaagacact gagacaccaa ttgacaccac cgtcatggca aaaagtgagg ttttctgcgt 8040
ccaaccagag aaaggaggcc gcaagccagc tcgccttatc gtattcccag acttgggggt 8100
tcgtgtatgc gagaagatgg ccctttatga cgtggtctcc acccttcctc aggccgtgat 8160
gggctcctca tacggattcc agtactcccc tggacagcgg gtcgagttcc tggtgaatgc 8220
ctggaaatca aagaaatgcc ctatgggctt ttcatatgac acccgctgtt ttgactcgac 8280
agtcactgag agtgacatcc gtgttgagga gtcaatttac caatgttgtg acttggcccc 8340
cgaagccaga caggccataa agtcgctcac agagcggctt tacattgggg gtcccctgac 8400
caattcaaaa gggcagaact gtggctatcg ccggtgccgc gcgagtggcg tgctgacgac 8460
cagctgcggt aataccctta catgttactt gaaggcctct gcagcctgtc gagctgcaaa 8520

gctccgggac tgcacgatgc tcgtgaacgg agacgacctc gtcgtcatct gtgagagtgc 8580
gggaacccaa gaggatgagg cgaacctacg agtcttcacg gaggctatga ctaggtattc 8640
tgcccccccc ggggacccgc cccgaccaga atacgacttg gagctaataa catcatgttc 8700
ctccaatgtg tcggtcgcgc acgatgcatc tggcaaaagg gtatactacc tcacccgcga 8760
cccctccacc ccccttgcac gggctgcgtg ggagacagct agacacactc cagttaattc 8820
ctggctaggc aacatcatta tgtatgcgcc caccttatgg gcaaggatga ttctgatgac 8880
ccatttcttc tccatccttc tagcccagga gcaacttgaa aaagccctgg attgccagat 8940
ctacggggcc tgttactcca ttgagccact tgacctacct cagatcattg aacgactcca 9000
tggtcttagc gcattttcac tccatagtta ctctccaggt gagatcaata gggtggcttc 9060
atgcctcagg aaacttgggg taccaccctt gcgagtctgg agacatcggg ccagaagtgt 9120
ccgcgctaag ctgctgtccc agggggggag ggctgccact tgtggtaagt acctcttcaa 9180
ctgggcagta aggaccaagc tcaaactcac tccaatcccg gcagcgtccc agttggactt 9240
gtccagctgg ttcgtggctg gttacagcgg gggagacata tatcacagcc tgtctcgtgc 9300
ccgaccccgc tggttcatgt tgtgcctact cctactttca gtaggggtag gcatctacct 9360
gctccccaac cgataaacgg ggagctaaac actccaggcc aataggccat ttcttttttt 9420
tttttttttt tttttctttt tttttttttt tttttttttt tttttttttt tttttttttt 9480
ctttcttttg tttttttttt ttttcttctt tttggtggct ccatcttagc cctagtcacg 9540
gctagctgtg aaaggtccgt gagccgcatg actgcagaga gtgctgatac tggcctctct 9600
gcagatcatg t 9611

## Claims

1. An oligoribonucleotide or peptide nucleic acid which sequence-specifically binds to the RNA of a hepatitis C virus (HCV) comprising the nucleotide sequence shown in SEQ ID No. 23 and having a chain length of 19 to 23 bases or 19 to 23 bp.

2. The oligoribonucleotide or peptide nucleic acid according to Claim 1 which hybridizes with the RNA of HCV under stringent conditions.

3. The oligoribonucleotide or peptide nucleic acid according to Claim 1 **characterized in that** the oligoribonucleotide or peptide nucleic acid hybridizes with the sequence of a 5' non-coding region of the RNA of HCV.

4. The oligoribonucleotide or peptide nucleic acid according to Claim 1 **characterized in that** the oligoribonucleotide or peptide nucleic acid hybridizes with the sequence of a highly identical region of the genetic sequences of a plurality of types of HCV different in genotype.

5. The oligoribonucleotide or peptide nucleic acid according to Claim 1 which is a donble-stranded RNA.

6. An oligoribonucleotide which hybridizes under stringent conditions either with an RNA region of HCV having a sequence complementary to the oligoribonucleotide according to Claim 1 or an RNA region of HCV hybridizing under stringent conditions with said oligoribonucleotide.

7. A siRNA consisting of either (a) the nucleotide sequence shown in SEQ ID No. 23 or (b) the nucleotide sequence shown in SEQ ID No. 23 wherein 7 or less nucleotides are deleted, substituted or added and which can inhibit HCV replication by hybridizing with the RNA of HCV.

8. A double-stranded siRNA comprising the siRNA according to Claim 7 as one of two strands, and which can inhibit HCV replication by hybridizing with the RNA of HCV.

9. A vector which expresses the oligoribonucleotide according to any one of Claims 1 to 6, the siRNA according to Claim 7, or the double-straaded siRNA according to Claim 8.

10. A therapeutic agent for hepatitis C containing as an active ingredient the oligoribonucleotide or peptide nucleic acid according to any one of Claims 1 to 6, the siRNA according to Claim 7, the double-stranded siRNA according to Claim 8, or the vector according to Claim 9.

11. A method of inhibiting replication, ability of HCV by allowing the oligoribonucleotide or peptide nucleic acid according to any one of Claims 1 to 6, the siRNA according to Claim 7, or the double-stranded siRNA according to Claim 8 to bind to the HVI-RNA.
